# EUROPEAN PATENT SPECIFICATION

(11) **EP 2 657 342 B1**
(45) Date of publication and mention of the grant of the patent: **18.11.2015**
(21) Application number: 13178199.9
(22) Date of filing: 10.08.2009
(51) Int. Cl.: C12N 15/87, C12N 15/88, A61K 48/00

(54) **DNA dendrimers protected against nuclease degradation**
Vor Nukleaseabbruch geschützte DNA-Dendrimere
Dendrimères d'ADN protégés contre la dégradation par les nucléases

(30) Priority: 08.08.2008 US 188318 P
(43) Date of publication of application: 30.10.2013
(62) Divisional of application: 09805654.2
(73) Proprietor: Genisphere, LLC, Hatfield, PA 19440 (US)
(72) Inventor: Kadushin, James M., Gilbertsville, PA 19525 (US); Getts, Robert C., Collegeville, PA 19426 (US)
(74) Representative: Herzog, Fiesser & Partner Patentanwälte PartG mbB

(56) References cited:
- Kadushin J.M. & Getts L.A.: "Enhancement of sensitivity in Luminex protein detection assays via dendrimer dependent signal amplification", Luminex Planet xMAP conference, April 25-27, 2005 (Austin TX). , 2005, XP002617108, Retrieved from the Internet: URL:http://www.genisphere.com/pdf/Genisphe re_Luminex_Planet_xMAP_042505.pdf [retrieved on 2011-01-17]
- Genisphere: "Starfish (TM) fluorescent in situ detection kit", , 2000, XP002617103, Retrieved from the Internet: URL:www.genisphere.com [retrieved on 2011-01-17]
- PATIL M.L. ET AL.: "Surface-modified and internally cationic polyamidoamine dendrimers for efficient siRNA delivery", BIOCONJUGATE CHEM., vol. 19, 25 June 2008 (2008-06-25), pages 1396-1403, XP002617104,
- Genisphere: An introduction to 3DNA technology , 2005, XP002617105, Retrieved from the Internet: URL:www.genisphere.com [retrieved on 2011-01-17]
- Dirk Haussecker: "Is dendrimer-siRNA delivery reaching critical mass?", Blog , 30 September 2009 (2009-09-30), XP002617106, Retrieved from the Internet: URL:http://rnaitherapeutics.blogspot.com/2 009/09/is-dendrimer-sirna-delivery-reachin g.html [retrieved on 2010-01-17]

## Description

### FIELD OF INVENTION

The invention is directed to the stabilization of DNA dendrimers against nuclease degradation in bodily fluids.

### BACKGROUND OF THE INVENTION

Dendritic molecules are repeatedly branched species that are characterized by structural perfection. This is based on the evaluation of both symmetry and polydispersity. The field of dendritic molecules can roughly be divided into low-molecular weight and high-molecular weight species. The first category includes dendrimers and dendrons, and the second includes dendronised polymers, hyperbranched polymers and brush-polymers.

The first dendrimers were synthesized divergently and in 1990 a convergent synthesis was introduced. Dendrimers then experienced an explosion of scientific interest because of their unique molecular architecture.

DNA dendrimers are complex, highly branched molecules built from interconnected natural or synthetic DNA subunits. A DNA dendrimer is constructed from partially double stranded DNA monomers, each of which is made from two single stranded DNA molecules that share a region of sequence complementarity located in the central portion of each strand. Monomers are combined during the manufacturing process to prepare DNA dendrimers of different sizes and shapes. In order to prevent DNA dendrimers from falling apart over time, chemical "spot welds" are added to the growing assembly during the process using UV light via the intercalation and activation of psoralen cross-linkers.

Multi-molecular scaffold devices, including DNA dendrimers, may be useful as cellular transfection, imaging, and drug delivery agents. Specifically, DNA dendrimers are bound with targeting devices (e.g. an antibody specific for a cell surface feature capable of eliciting an cellular endocytotic internalization event) and can bind to surface features on cells targeted to receive the delivery of a cargo (e.g. a drug). Cargos may be passively associated with the targeted DNA dendrimer and enter the cell simply by spatial association with the dendrimer, or cargos may be directly bound to the dendrimer via a number of attachment strategies.

The typical response elicited by siRNA molecules is referred to as mRNA knockdown or reduction of steady-state mRNA levels, with the sequence of the siRNA molecule determining which gene or genes are to be targeted for knockdown.

### SUMMARY OF THE INVENTION

The present invention is directed to the use of a compound selected from the group consisting of a protein, digoxigenin, cholesterol, a primary amine, a hydrocarbon spacer of length of 3 to 120 carbons, a PEG molecule, biotin or a biotin derivative, fluorescein or a fluorescein derivative, or any combination thereof, for protecting a DNA dendrimer against nuclease degradation in a bodily fluid, wherein the compound is attached to the dendrimer.

### BRIEF DESCRIPTION OF THE DRAWINGS

FIG. 1 is an illustration of the DNA strands and monomers used for preparation of dendrimer components.
FIG. 2 is an illustration of the DNA dendrimer assembly steps, showing the sequential growth of the DNA dendrimers (as layers) as various monomers are added.

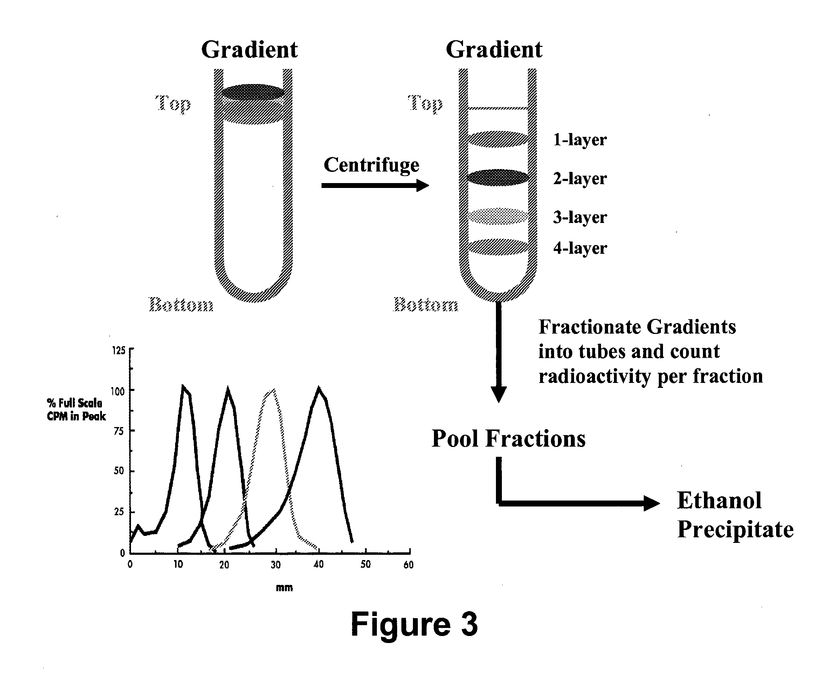

FIG. 3 is an illustration of DNA dendrimers' purification steps.

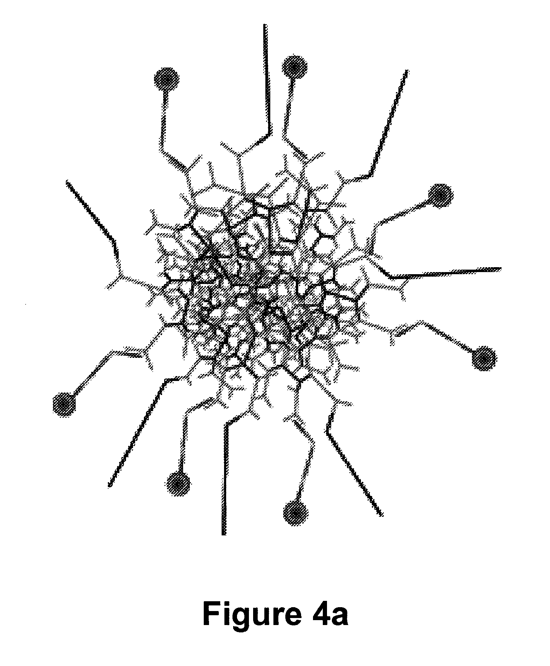

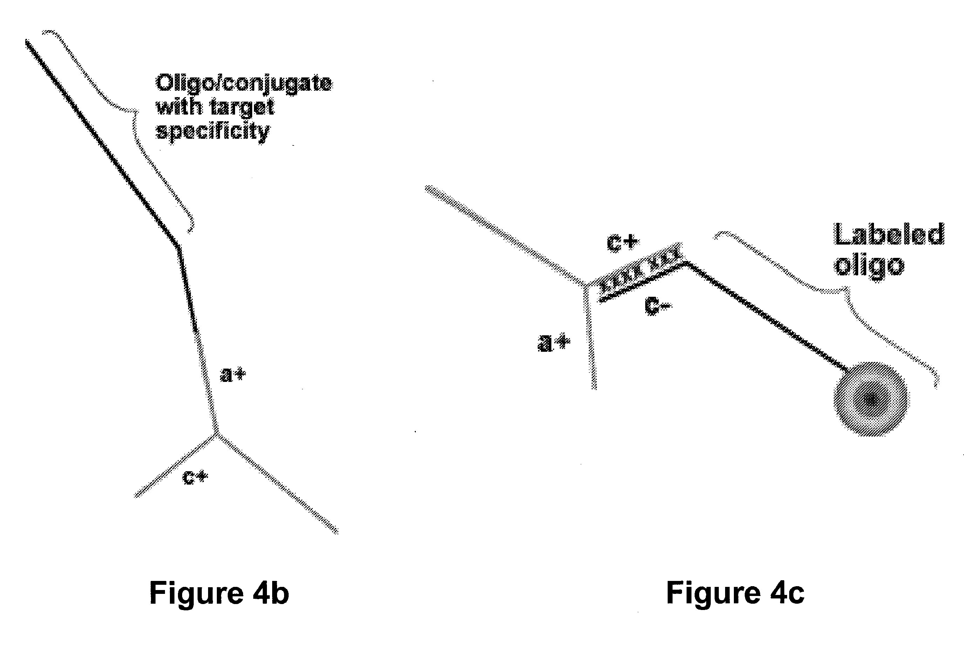

FIG. 4 is an illustration of DNA dendrimers comprising attachments of specific oligo and signal molecules (4a); enlarged image of the target specificity bound to the dendrimer via oligo ligation (4b); and enlarged image of the covalent attachment of a labeled oligo to the arm of the dendrimer (4c).
FIG. 5 is a micrograph of a gel showing the degradation of: the non-modified four layer DNA dendrimer, 0-120min (5a); and the modified four layer DNA dendrimer 0-120min (resistant) (5b).
FIG. 6 is a micrograph of a gel showing the degradation of: the non-modified four layer DNA dendrimer 0-960min (6a); and the modified four layer DNA dendrimer 0-960 minutes (resistant) (6b).

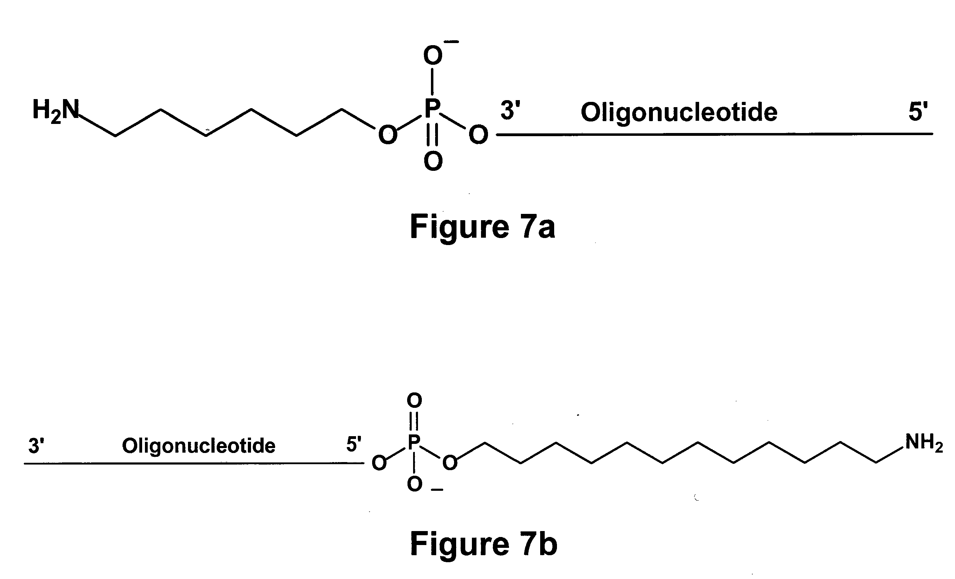

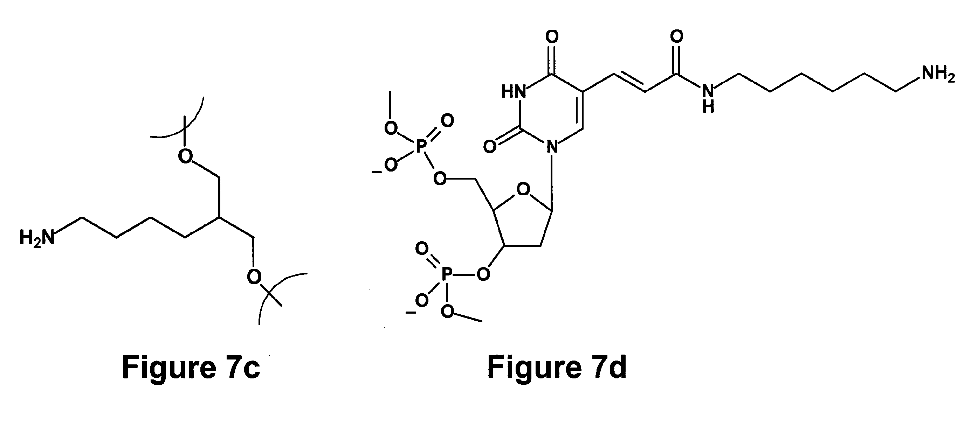

FIG. 7. Chemical structures of: 6 carbon amino linker amidite structure (for DNA oligo synthesis) (7A); 12 carbon amino linker amidite structure (for DNA oligo synthesis) (7B); 7 carbon internal amino linker amidite structure (for DNA oligo synthesis) (7C); dT internal amino linker amidite structure (for DNA oligo synthesis) (7D).
FIG. 8. Chemical structures of NHS esters; Cy™3 NHS ester (8a) Excitation max = 548 nm, Emission max = 562 nm; Cy5 NHS ester (8b) Excitation max = 646 nm, Emission max = 664 nm.
FIG 9. Chemical structure of Oyster 550-D (9a); and Chemical structure of Oyster 650-D (9b).

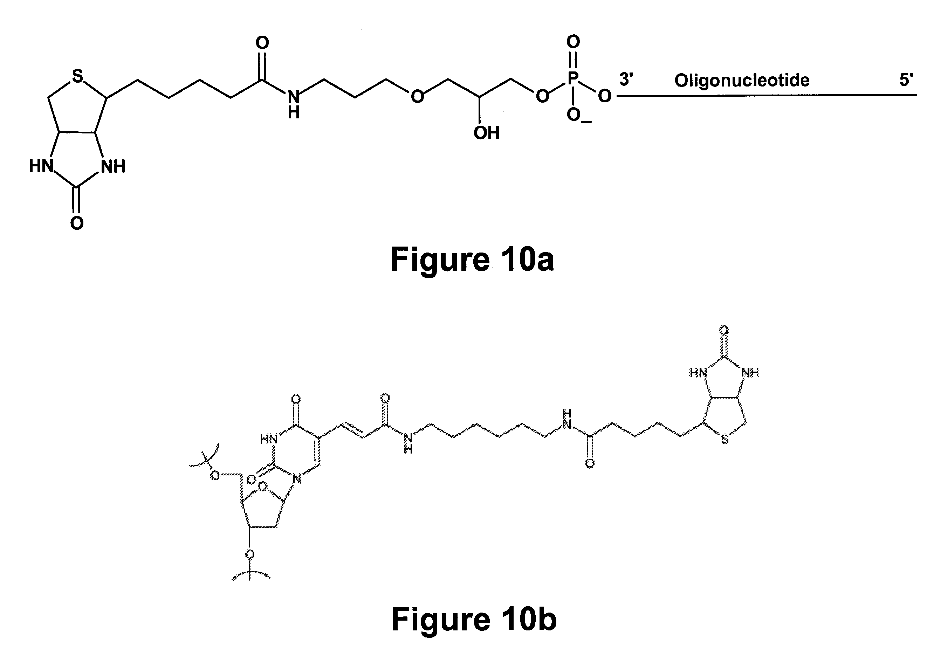

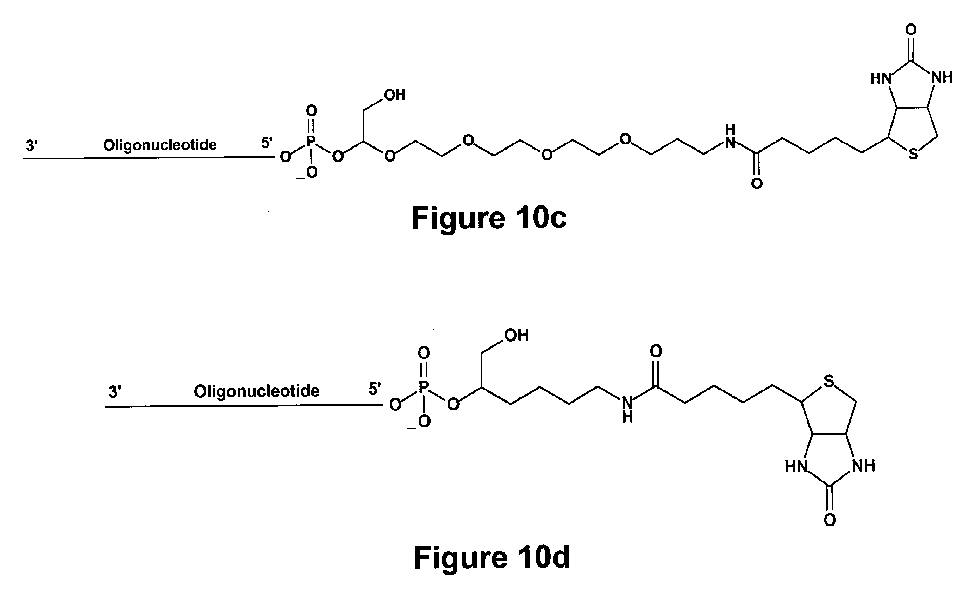

FIG. 10. Chemical structures of: Biotin-BB-CPG amidite structure (for DNA oligo synthesis) (10a); Structure of biotin-dT amidite (for DNA oligo synthesis) (10b); Structure of biotin-TEG amidite (for DNA oligo synthesis) C9O4 spacer (10c); Structure of biotin-TEG amidite (for DNA oligo synthesis) C4 spacer (10d).

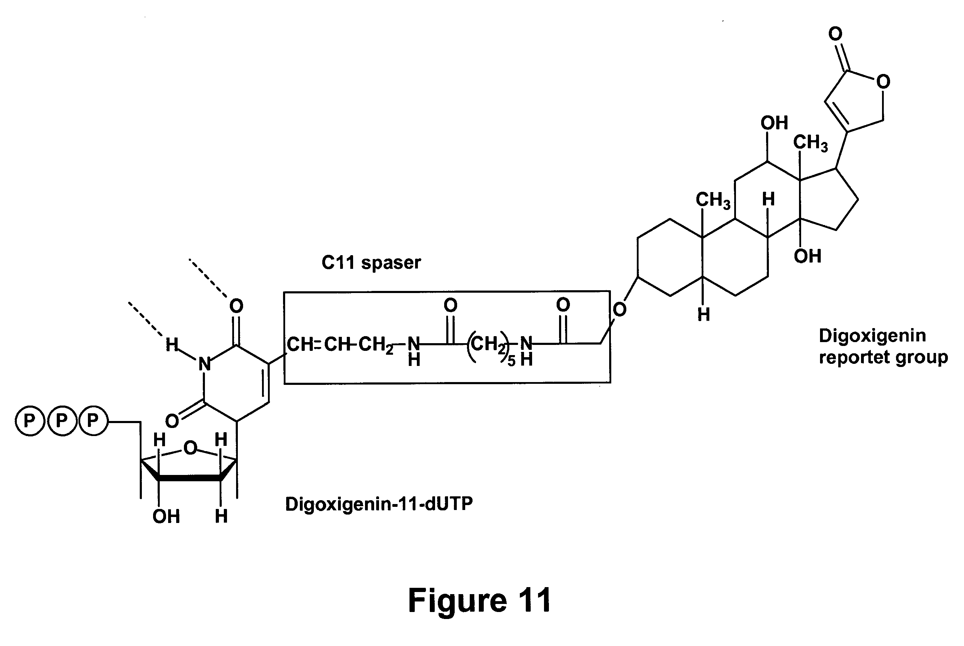

FIG 11. Chemical structures of digoxigenin 11-UTP and the reporter.

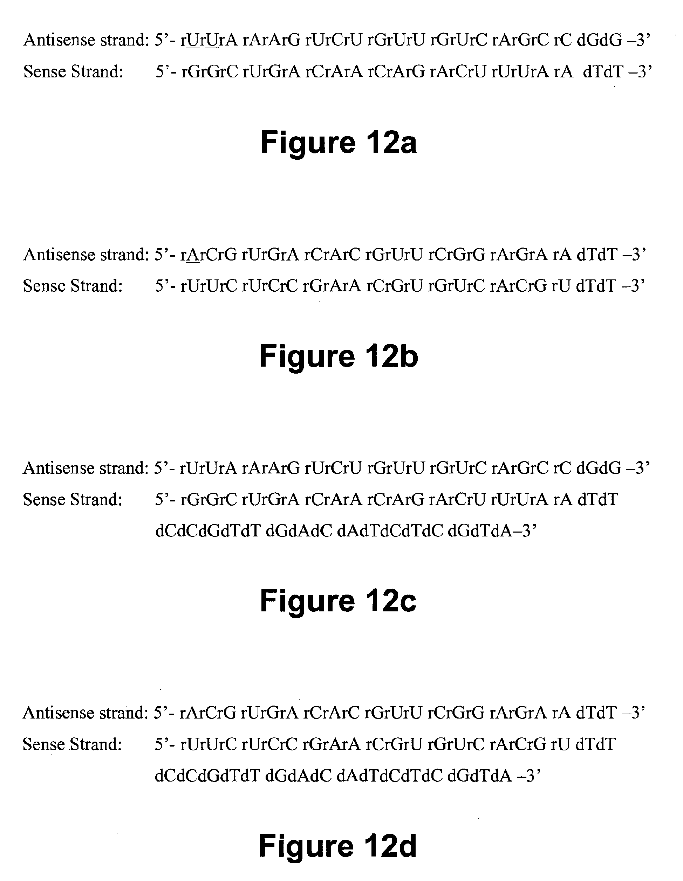

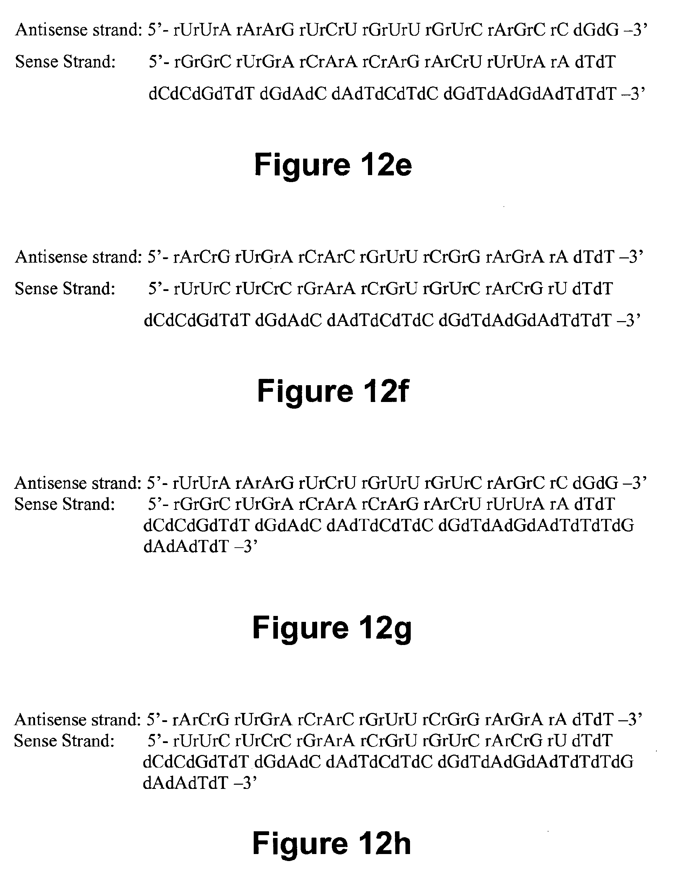

FIG 12. The siRNA RNA-DNA hybrid constructs utilized with the DNA dendrimers: the sequences of the antisense and sense strands of the SSB siRNAs (12a) and the negative control siRNA (12b); the sequences of the antisense and sense strands of the SSB with 16 base DNA linker sequence (12c) and the negative control (no mRNA target) with 16 base linker sequence (12d); the sequences of the antisense and sense strands of the SSB with 21 base DNA linker sequence (12e) and the negative control (no mRNA target) with 21 base linker sequence (12f); and the sequences of the antisense and sense strands of the SSB with 26 base DNA linker sequence (12g) and the negative control (no mRNA target) with 26 base linker sequence (12h).

### DETAILED DESCRIPTION OF THE INVENTION

Described herein is a composition comprising a DNA dendrimer attached to a siRNA molecule. The composition may further comprises a protein, a fluorescein or a fluorescein derivative such as but not limited to FITC, a PEG molecule, biotin, a biotin derivative, or any combination thereof further attached to the DNA dendrimer. Surprisingly, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, a FITC, a PEG molecule, biotin, a biotin derivative, or any combination thereof to a DNA dendrimer protected the DNA dendrimer from body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof to a DNA dendrimer protected the DNA dendrimer and the siRNA molecule attached thereto from body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein or fluorescein derivative, or any combination thereof to a DNA dendrimer, protected the DNA dendrimer and the siRNA molecule attached thereto from body fluid and serum degradation. For another surprising observation, it was found that attaching a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein or fluorescein derivative, or any combination thereof to a DNA dendrimer protected the DNA dendrimer and the siRNA molecule attached thereto from nuclease dependent degradation.

In an embodiment of the invention, the nuclease is a protein DNase. In another embodiment of the invention, the nuclease is an exogenous DNase. In another embodiment of the invention, the nuclease may be any different protein DNases known to one of skill in the art. In another aspect, serum degradation may be serum nuclease degradation. Surprisingly, it was found that attaching a protein, a FITC, a PEG molecule, biotin, a biotin derivative, fluorescein or a fluorescein derivative, or any combination thereof to a DNA dendrimer stabilizes the DNA dendrimer and the siRNA molecule attached thereto against serum degradation.

The composition may comprise a protective group, which protects the composition against degradation. The composition may comprise a protective group, which protects the composition against degradation in body fluids such as serum, blood plasma, etc. The composition may comprise a protective group, which protects the composition against nuclease dependent degradation. A protective group may be a compound or a molecule, which stabilizes the DNA dendrimer. A protective group may be a compound or a molecule which protects the DNA dendrimer against degradation. A protective group may be a compound or a molecule which protects the DNA dendrimer against degradation in a body fluid. A protective group may be a compound or a molecule which protects the DNA dendrimer against degradation in serum.

One stabilized composition as described herein is stable in serum, in a composition comprising serum, in blood, or any other body fluid for at least 0.3 hours. Another one stabilized composition as described herein is stable in serum, in a composition comprising serum, in blood, or any other body fluid for at least 0.5 hours. Another one stabilized composition as described herein is stable in serum, in a composition comprising serum, in blood, or any other body fluid for at least 0.7 hours. Another one stabilized composition as described herein is stable in serum, in a composition comprising serum, in blood, or any other body fluid for at least 1 hour. Another one stabilized composition as described herein is stable in serum, in a composition comprising serum, in blood, or any other body fluid for at least 1.5 hours. Another one stabilized composition as described herein is stable in serum, in a composition comprising serum, in blood, or any other body fluid for at least 2 hours. Another one stabilized composition as described herein is stable in serum, in a composition comprising serum, in blood, or any other body fluid for at least 3 hours. Another one stabilized composition as described herein is stable in serum, in a composition comprising serum, in blood, or any other body fluid for at least 4 hours. Another one stabilized composition as described herein is stable in serum, in a composition comprising serum, in blood, or any other body fluid for at least 5 hours. Another one stabilized composition as described herein is stable in serum, in a composition comprising serum, in blood, or any other body fluid for at least 6 hours. Another one stabilized composition as described herein is stable in serum, in a composition comprising serum, in blood, or any other body fluid for at least 7 hours. Another one stabilized composition as described herein is stable in serum, in a composition comprising serum, in blood, or any other body fluid for at least 8 hours. Another one stabilized composition as described herein is stable in serum, in a composition comprising serum, in blood, or any other body fluid for at least 9 hours. Another one stabilized composition as described herein is stable in serum, in a composition comprising serum, in blood, or any other body fluid for at least 10 hours. Another one stabilized composition as described herein is stable in serum, in a composition comprising serum, in blood, or any other body fluid for at least 11 hours. Another one stabilized composition as described herein is stable in serum, in a composition comprising serum, in blood, or any other body fluid for at least 12 hours. Another one stabilized composition as described herein is stable in serum, in a composition comprising serum, in blood, or any other body fluid for at least 2 hours. Another one stabilized composition as described herein is stable in serum, in a composition comprising serum, in blood, or any other body fluid for at least 13 hours. Another one stabilized composition as described herein is stable in serum, in a composition comprising serum, in blood, or any other body fluid for at least 14 hours. Another one stabilized composition as described herein is stable in serum, in a composition comprising serum, in blood, or any other body fluid for at least 15 hours. Another one stabilized composition as described herein is stable in serum, in a composition comprising serum, in blood, or any other body fluid for at least 16 hours. Another one stabilized composition as described herein is stable in serum, in a composition comprising serum, in blood, or any other body fluid for at least 20 hours. Another one stabilized composition as described herein is stable in serum, in a composition comprising serum, in blood, or any other body fluid for at least 24 hours. Another one stabilized composition as described herein is stable in serum, in a composition comprising serum, in blood, or any other body fluid for at least 30 hours. Another one stabilized composition as described herein is stable in serum, in a composition comprising serum, in blood, or any other body fluid for at least 36 hours. Another one stabilized composition as described herein is stable in serum, in a composition comprising serum, in blood, or any other body fluid for at least 48 hours. Another one stabilized composition as described herein is stable in serum, in a composition comprising serum, in blood, or any other body fluid for at least 60 hours. Another one stabilized composition as described herein is stable in serum, in a composition comprising serum, in blood, or any other body fluid for at least 72 hours.

Another one stabilized composition as described herein is stable in serum, in a composition comprising serum, in blood, or any other body fluid for 1-24 hours. Another one stabilized composition as described herein is stable in serum, in a composition comprising serum, in blood, or in any other body fluid for 1-20 hours. Another one stabilized composition as described herein is stable in serum, in a composition comprising serum, in blood, or any other body fluid for 5-15 hours. Another one stabilized composition as described herein is stable in serum, in a composition comprising serum, in blood, or any other body fluid for 10-16 hours.

Further described herein is a composition comprising an antibody or a fragment thereof and a siRNA molecule attached to a DNA dendrimer. The antibody may be a conjugated antibody, a monoclonal antibody, a polyclonal antibody, is a single-chain Fv fragment (SCFV) antibody or any antibody or a conjugated antibody known to one of skill in the art.

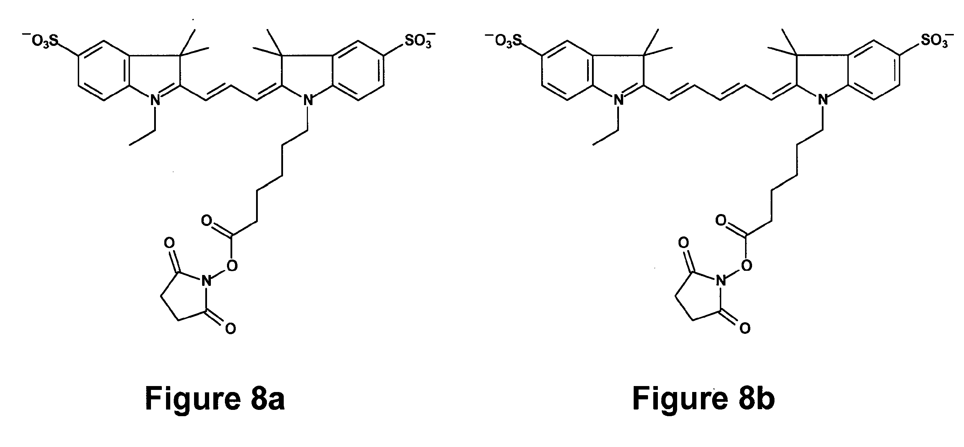

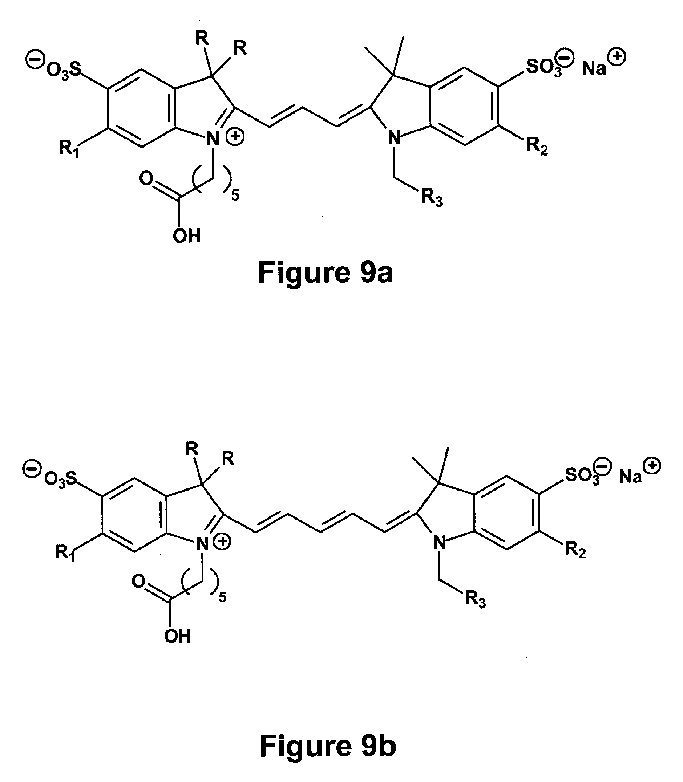

Further described herein is a composition comprising a dye molecule and a siRNA molecule attached to a DNA dendrimer. Also described herein is a composition comprising a dye molecule comprising a detectable label attached to a linker or spacer (figure 8 and figure 9) and a siRNA molecule attached to a DNA dendrimer. Also described herein is a composition comprising a fluorophore and a siRNA molecule attached to a DNA dendrimer. Also described herein is a composition comprising fluorescein and a siRNA molecule attached to a DNA dendrimer. Also described herein is a composition comprising fluorescein isothiocyanate (FITC) and a siRNA molecule attached to a DNA dendrimer. FITC is referred as a fluorescein derivative.

Further described herein is a composition comprising a molecule comprising an ureido (tetrahydroimidizalone) ring and a siRNA molecule attached to a DNA dendrimer. Also described herein is a composition comprising a molecule comprising a tetrahydrothiophene ring and a siRNA molecule attached to a DNA dendrimer. Also described herein is a composition comprising a molecule comprising valeric acid substituent and a siRNA molecule attached to a DNA dendrimer. Also described herein is a composition comprising a molecule serving as a cofactor in the metabolism of fatty acids and a siRNA molecule attached to a DNA dendrimer. Also described herein is a composition comprising a molecule serving as a cofactor in the metabolism of leucine and a siRNA molecule attached to a DNA dendrimer. Also described herein is a composition comprising biotin and a siRNA molecule attached to a DNA dendrimer.

The term "biotin" may include known biotin derivatives. A biotin derivative may bind strepavidin. The term "fluorescein," may include known fluorescein derivatives.

Also a composition is described herein, further comprising serum. Also a composition is described herein, further comprising blood. Also a composition is described herein further comprising antibodies, electrolytes and soluble proteins. Another composition as described herein further comprises a cationic agent.

Further described herein is a method for preparing a composition comprising a DNA dendrimer and a siRNA molecule, comprising the step of attaching the siRNA to the DNA dendrimer (a) via a disulfide bridging bond; (b) via the use of *N*-hydroxysuccinimide (NHS) ester dependent condensation reaction; (c) via the use of bifunctional cross linking reaction; (d) via direct or indirect hybridization of the siRNA to DNA dendrimer sequence; or (e) via the use of polycationic compounds to bridge siRNA to a DNA dendrimer via charge-charge interactions. Also described herein is a method further comprising the step of attaching to the DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof.

Further described herein is a method of protecting a siRNA molecule against degradation, comprising the step of attaching (a) a siRNA molecule and (b) a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof to a DNA dendrimer, thereby protecting a siRNA molecule against degradation. The attachment of protein, a FITC, a PEG molecule, biotin, a biotin derivative, or any combination thereof to the DNA dendrimer stabilized the DNA dendrimer and the siRNA molecule attached thereto. The attachment of protein, a FITC, a PEG molecule, biotin, a biotin derivative, fluorescein, or any combination thereof to the DNA dendrimer unexpectedly stabilizes the DNA dendrimer and the siRNA molecule attached thereto. The attachment of protein, a FITC, a PEG molecule, biotin, a biotin derivative, fluorescein, or any combination thereof to the DNA dendrimer unexpectedly stabilizes the DNA dendrimer and the siRNA molecule attached thereto against serum degradation. The attachment of an antibody or a fragment thereof to the DNA dendrimer unexpectedly stabilizes the DNA dendrimer and the siRNA molecule attached thereto against serum degradation. The attachment of biotin to the DNA dendrimer unexpectedly stabilizes the DNA dendrimer and the siRNA molecule attached thereto against serum degradation. The attachment of PEG to the DNA dendrimer unexpectedly stabilizes the DNA dendrimer and the siRNA molecule attached thereto against serum degradation. The attachment of FITC to the DNA dendrimer unexpectedly stabilizes the DNA dendrimer and the siRNA molecule attached thereto against serum degradation. The attachment of fluorescein to the DNA dendrimer unexpectedly stabilizes the DNA dendrimer and the siRNA molecule attached thereto against serum degradation.

Further described herein is a method of protecting a siRNA molecule against degradation comprising the step of attaching to the DNA dendrimer a molecule comprising a DNA molecule, a RNA molecule, a protein, a peptide, a chemotherapeutic agent, an antiviral agent, an anti-inflammatory agent, a bacteriostatic agent, a psychoactive agent, a statin, a neuropathic agents, a hormone, an ACE inhibitor, an anti-clotting factor, an analgestic, an anti- angiogenic agent, a pro-angiogenic agent, a growth factor, a growth factor inhibitor, or any combination thereof.

The present invention is directed to the use of a compound selected from the group consisting of a protein, a PEG molecule, biotin or a biotin derivative, fluorescein or a fluorescein derivative, digoxigenin, cholesterol, primary amine, a hydrocarbon spacer of length 3 to 120 carbons, or any combination thereof, for protecting a DNA dendrimer against nuclease degradation in a bodily fluid, wherein the compound is attached to the dendrimer.

Further described herein is a method of preparing a composition comprising a DNA dendrimer and a DNA molecule, a RNA molecule, a protein, a peptide, a chemotherapeutic agent, an antiviral agent, an anti-inflammatory agent, a bacteriostatic agent, a psychoactive agent, a statin, a neuropathic agents, a hormone, an ACE inhibitor, an anti-clotting factor, an analgestic, an anti- angiogenic agent, a pro-angiogenic agent, a growth factor, a growth factor inhibitor, or any combination thereof, comprising the step of attaching a siRNA to the DNA dendrimer (a) via a disulfide bridging bond; (b) via the use of NHS ester dependent condensation reaction; (c) via the use of heterobifunctional cross linking reaction; (d) via direct or indirect hybridization of the siRNA to DNA dendrimer sequence; or (e) via the use of polycationic compounds to bridge siRNA to the DNA dendrimer via charge-charge interactions.

It was unexpectedly found that the attachment of low molecular weight fluorescent dyes (less than 10,000 daltons) to the DNA dendrimer stabilized a composition comprising a DNA dendrimer or a composition comprising a DNA dendrimer and a siRNA molecule attached thereto. It was unexpectedly found that the attachment of low molecular weight fluorescent dyes (less than 10,000 daltons), comprising a fluorescent molecule attached to a linker or spacer, to the DNA dendrimer stabilized a composition comprising a DNA dendrimer or a composition comprising a DNA dendrimer and a siRNA molecule attached thereto. The attachment of low molecular weight fluorescent dyes (less than 10,000 daltons) comprising a fluorescent molecule attached to a linker or spacer to the DNA dendrimer stabilizes a composition comprising a DNA dendrimer or a composition comprising a DNA dendrimer and a siRNA molecule attached thereto. The attachment of cyanine dyes such as but not limited to Cy3 or Cy5 (Figure 8) to the DNA dendrimer unexpectedly stabilizes a composition comprising a DNA dendrimer or a composition comprising a DNA dendrimer and a siRNA molecule attached thereto. The attachment of Oyster dyes comprising an Oyster dye molecule attached to a linker or spacer, such as but not limited to Oyster 550 or Oyster 650 (Figure 9) to the DNA dendrimer unexpectedly stabilizes a composition comprising a DNA dendrimer or a composition comprising a DNA dendrimer and a siRNA molecule attached thereto. The attachment of Alexa Fluor dyes, comprising an Alexa Fluor molecule attached to a linker or spacer, such as but not limited to Alexa Fluor 350, Alexa Fluor 488, Alexa Fluor 532, Alexa Fluor 546, Alexa Fluor 555, Alexa Fluor 594, Alexa Fluor 633, Alexa Fluor 647, Alexa Fluor 655, Alexa Fluor 660, Alexa Fluor 680, or Alexa Fluor 700 to the DNA dendrimer unexpectedly stabilizes a composition comprising a DNA dendrimer or a composition comprising a DNA dendrimer and a siRNA molecule attached thereto. The attachment of fluorescein comprising an fluoroscein molecule attached to a linker or spacer, and derivatives such as but not limited to FITC, 5/6-carboxyfluorescein succinimidyl ester, 5/6-FAM SE, or 5(6) fluorescein isothiocyanate mixed isomer, 5/6-FITC to the DNA dendrimer unexpectedly stabilizes a composition comprising a DNA dendrimer or a composition comprising a DNA dendrimer and a siRNA molecule attached thereto. The attachment of BODIPY 630/650 comprising a BODIPY molecule attached to a linker or spacer, to the DNA dendrimer unexpectedly stabilizes a composition comprising a DNA dendrimer or a composition comprising a DNA dendrimer and a siRNA molecule attached thereto.

The attachment of high molecular weight fluorescent dyes (greater than 50,000 daltons) such as but not limited to R-Phycoerythrin (conjugated to streptavidin), bound to biotin previously incorporated into the DNA dendrimer structure), B-Phycoerythrin (conjugated to streptavidin), bound to biotin previously incorporated into the DNA dendrimer structure), or Allophycocyanin (APC) (conjugated to streptavidin), bound to biotin previously incorporated into the DNA dendrimer structure) to the DNA dendrimer unexpectedly stabilizes a composition comprising a DNA dendrimer or a composition comprising a DNA dendrimer and a siRNA molecule attached thereto.

The attachment of non-fluorescent low molecular weight compounds such as but not limited to biotin and derivatives, NHS-biotin (for covalent binding to primary amines previously incorporated into the DNA dendrimer or oligonucleotide structure), Biotin-BB-CPG amidite (for synthesis of synthetic DNA oligonucleotides, Biotin-dT amidite amidite (for synthesis of synthetic DNA oligonucleotides, Biotin-BB-CPG amidite (for synthesis of synthetic DNA oligonucleotides), or digoxigenin derivatives to the DNA dendrimer unexpectedly stabilizes a composition comprising a DNA dendrimer or a composition comprising a DNA dendrimer and a siRNA molecule attached thereto.

The attachment of oligonucleitides, comprising Cholesterol TEG 5' / 3' amidite, propyl 3' spacer amidite, 12 carbon spacer amidite, 18 carbon spacer amidite, 6 carbon amino linker amidite, 12 carbon amino linker amidite, 7 carbon internal amino linker amidite, or dT internal amino linker amidite to the DNA dendrimer unexpectedly protects a composition comprising a DNA dendrimer or a composition comprising a DNA dendrimer and a siRNA molecule attached thereto from degradation.

Cyanine, Oyster and Alexa Fluor dyes have been incorporated into the DNA dendrimer structure as: NHS-ester dye conjugates covalently bound to primary amines located either directly on the DNA dendrimer structure or on synthetic oligonucleotides subsequently hybridized and crosslinked to the DNA dendrimer structure. DNA and/or RNA nucleotides have been incorporated during synthetic oligonucleotide synthesis (as labeled amidites) or via the use of RNA or DNA polymerases incorporating labeled RNA and/or DNA nucleotides (ribonucleotides and/or deoxyribonucleotides). Streptavidin conjugates that bind to biotin have been previously incorporated into the DNA dendrimer structure.

The present invention is directed to the use of a compound selected from the group consisting of a protein, fluorescein, a fluorescein derivative, digoxigenin, cholesterol, a primary amine, a hydrocarbon spacer of length 3 to 120 carbons, a PEG molecule, biotin, a biotin derivative, or any combination thereof, for protecting a DNA dendrimer against serum nuclease degradation or nuclease degradation in a bodily fluid, wherein the compound is attached to the dendrimer. In another embodiment of the invention, for protecting a DNA dendrimer against serum or body fluid degradation, an antibody or a fragment thereof is attached to a DNA dendrimer. In another embodiment of the invention, for protecting a DNA dendrimer against serum or body fluid degradation, biotin or a biotin derivative is attached to a DNA dendrimer. In another aspect for protecting a DNA dendrimer against serum degradation, FITC is attached to a DNA dendrimer. In another embodiment of the invention, for protecting a DNA dendrimer against serum or body fluid degradation, fluorescein or a fluorescein derivative is attached to a DNA dendrimer.

Also described herein is a method of protecting a DNA dendrimer carrying a molecule comprising biological activity comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof, thereby protecting a DNA dendrimer carrying a molecule comprising biological activity against serum nuclease degradation. A molecule comprising biological activity may be an enzyme. A molecule comprising biological activity may be a therapeutic agent. A molecule comprising biological activity may be a chemotherapeutic agent. A molecule comprising biological activity may be a cytokine. A molecule comprising biological activity may be a chemokine. A molecule comprising biological activity may be a hormone. A molecule comprising biological activity may be a neurotransmitter. A molecule comprising biological activity may be a neurotransmitter precursor. A molecule comprising biological activity may be a neurotransmitter agonist. A molecule comprising biological activity may be a neurotransmitter antagonist. A molecule comprising biological activity may be a non-steroidial anitinflamatory agent. A molecule comprising biological activity may be a vitamin. A molecule comprising biological activity may be a clotting factors. A molecule comprising biological activity may be a chelator. A molecule comprising biological activity may be a peptide. A molecule comprising biological activity may be a protein. A molecule comprising biological activity may be a lipid.

Also described herein is a method of delivering a DNA dendrimer into a body fluid, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination, thereby delivering a DNA dendrimer into a serum. Also described herein is a method of delivering a DNA dendrimer into a body fluid, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination, thereby delivering a DNA dendrimer into a composition comprising serum or a body fluid. Also described herein is a method of delivering a DNA dendrimer into a serum or a body fluid, comprising the step of attaching to a DNA dendrimer an antibody or a fragment thereof, thereby delivering a DNA dendrimer into a composition comprising serum or a body fluid. Also described herein is a method of delivering a DNA dendrimer into a composition comprising serum or a body fluid, comprising the step of attaching to a DNA dendrimer biotin, thereby delivering a DNA dendrimer into a serum. Also described herein is a method of delivering a DNA dendrimer into a composition comprising serum or a body fluid, comprising the step of attaching to a DNA dendrimer a dye molecule, thereby delivering a DNA dendrimer a composition comprising serum or a body fluid. Also described herein is a method of delivering a DNA dendrimer into a serum, comprising the step of attaching to a DNA dendrimer a PEG, thereby delivering a DNA dendrimer a composition comprising serum or a body fluid.

Also described herein is a method of delivering a DNA dendrimer attached to a nucleic acid molecule into a serum, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, or any combination thereof. Also described herein is a method of delivering a DNA dendrimer attached to a siRNA molecule into a serum, comprising the step of attaching to a DNA dendrimer a protein, a peptide, an aptamer, fluorescein, a fluorescein derivative, a fluorescent dye, digoxigenin, cholesterol, a primary amine, a hydrocarbon spacer of length 3 to 120 carbons, FITC, a PEG molecule, biotin, a biotin derivative, fluorescein, or any combination thereof.

Delivering may be in vitro mixing or in vivo delivery. In vivo delivery of a composition as described herein or a DNA dendrimer modified with attachments as described is preformed by methods known to one of skill in the art such as but not limited to intravenous or intra-arterial injections.

Also described herein is a method of transfecting a cell with a siRNA molecule, comprising the step of contacting a cell with a composition comprising a DNA dendrimer and a siRNA molecule as described herein, thereby transfecting a cell with a siRNA molecule. Also described herein is a method of transfecting a cell with a siRNA molecule, comprising the step of contacting a cell with a composition comprising a DNA dendrimer, antibody or a fragment thereof, and a siRNA molecule as described herein, thereby transfecting a cell with a siRNA molecule. Also described herein is a method of transfecting a cell with a siRNA molecule, comprising the step of contacting a cell with a composition comprising a DNA dendrimer, biotin, and a siRNA molecule as described herein, thereby transfecting a cell with a siRNA molecule. Also described herein is a method of transfecting a cell with a siRNA molecule, comprising the step of contacting a cell with a composition comprising a DNA dendrimer, dye molecule, and a siRNA molecule as described herein, thereby transfecting a cell with a siRNA molecule.

In another embodiment of the invention, a nucleic acid molecule is further attached to the DNA dendrimer via a non covalent bond. In another embodiment of the invention, a nucleic acid molecule is attached to the DNA dendrimer via a covalent bond. In another embodiment of the invention, the nucleic acid molecule is siRNA. In another embodiment of the invention, a nucleic acid molecule is attached to the DNA dendrimer via hydrogen bonds such as Watson-Crick base pairing. In another embodiment of the invention, a nucleic acid molecule is attached to the DNA dendrimer via a disulfide bridging bond. In another embodiment of the invention, a nucleic acid molecule is attached to the DNA dendrimer via an NHS ester and an amine. In another embodiment of the invention, a nucleic acid molecule is attached to the DNA dendrimer via a heterobifunctional cross-linking bond. In another embodiment of the invention, the term "attachment" as used herein refers to a chemical bond.

In another embodiment of the invention, the DNA strands are covalently bonded in each layer of the dendrimer. In another embodiment of the invention, the DNA dendrimer comprises at least one Cap03 sequence located within the arm of the DNA dendrimer. In another embodiment the Cap03 sequence comprises the following nucleic acid sequence: 5'-TCCACCTTAgAgTACAAACggAACACgAgAA-3' (SEQ ID NO: 8). In another embodiment of the invention, the Cap03 sequence is used for binding/attaching a molecule such as an antibody or a fragment thereof comprising a Cap03 anti-sense sequence, to the DNA dendrimer. In another embodiment, the Cap03 anti-sense sequence comprises the following nucleic acid sequence: 5'-TTCTCgTgTTCCgTTTgTACTCTAAggTggA-3' (SEQ ID NO: 9). In another embodiment of the invention, methods of preparing a composition as described herein comprise the step of non-covalently binding a protein covalently conjugated to a DNA molecule comprising a sequence complementary to Cap03 sequence to the DNA dendrimer.

In another embodiment of the invention, the nucleic acid molecule comprises a binding moiety and the DNA dendrimer comprises a binding partner wherein the binding partner non-covalently binds to the binding moiety.

In another embodiment of the invention, a DNA dendrimer is covalently cross linked. In another embodiment of the invention, a DNA dendrimer is a four layer DNA dendrimer. In another embodiment of the invention, a DNA dendrimer is a two layer DNA dendrimer. In another embodiment, a DNA Dendrimer is any dendrimer prepared from 3 or more strands of DNA.

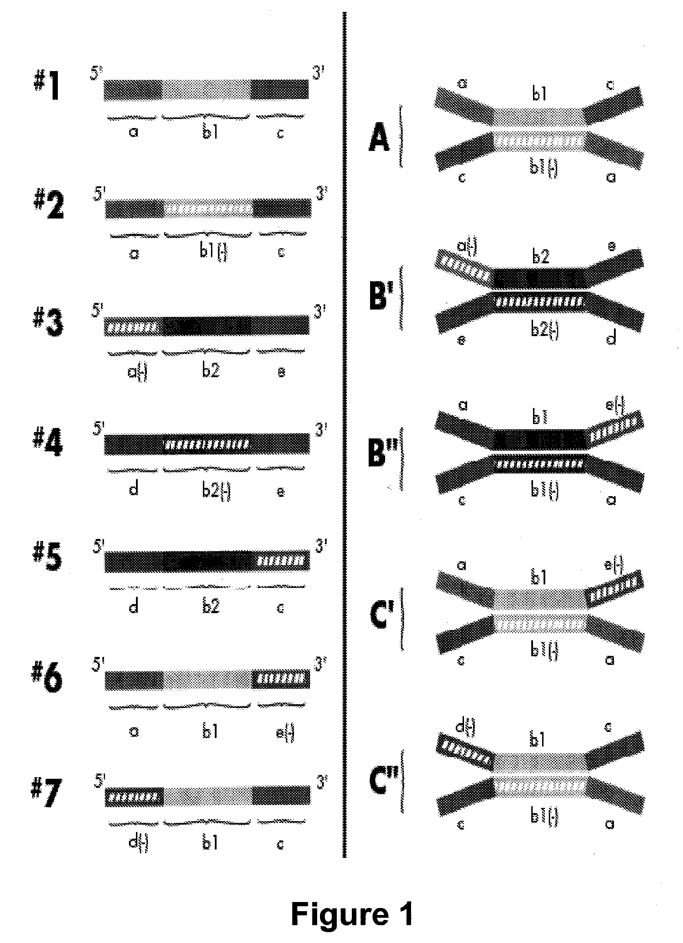

Further described herein are methods for the manufacture and use of a DNA dendrimer containing a targeting antibody or a fragment thereof and a siRNA molecule non-covalently bound via a hybridization event (example 1). In another embodiment of the invention, a DNA dendrimer as described herein is constructed from DNA monomers, each of which was made from two DNA strands that share a region of sequence complementarity located in the central portion of each strand. In another embodiment of the invention, a DNA dendrimer comprises a structure described as having a central doublestranded "waist" bordered by four single-stranded "arms". In another embodiment of the invention, a DNA dendrimer comprises a waist-plus-arms structure which includes the basic DNA monomer. In another embodiment, the single-stranded arms at the ends of each of the five monomer types interact with one another in precise and specific ways. In another embodiment of the invention, base-pairing (hydrogen bonding) allows directed assembly of the dendrimer through sequential addition of monomer layers (see Figure 1).

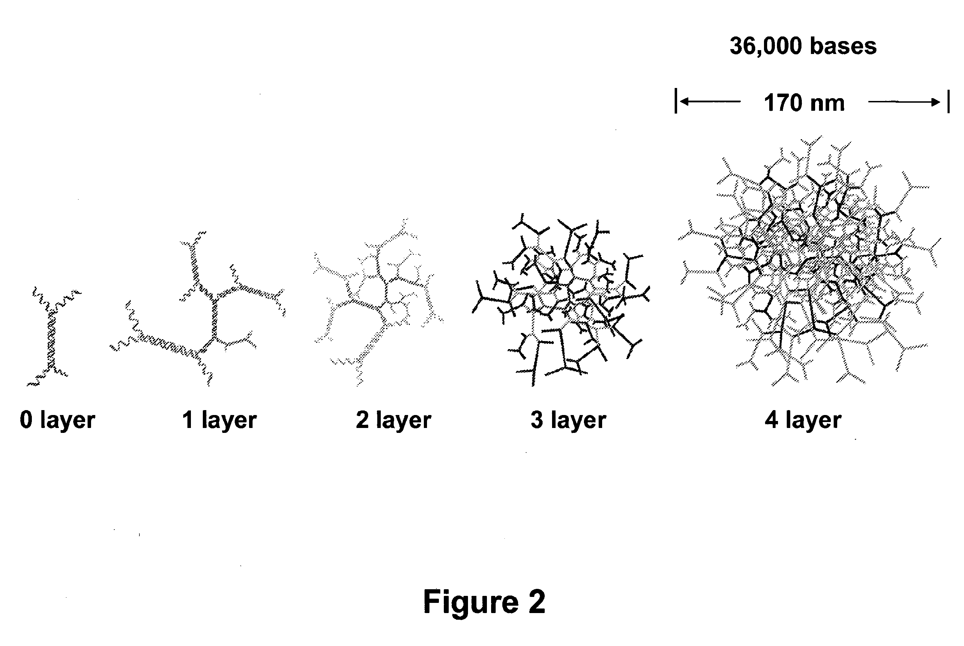

In another embodiment of the invention, a DNA dendrimer as described herein is assembled by a cross-linking process where the strands of DNA are covalently bonded to each other; thereby forming a completely covalent molecule impervious to denaturing conditions that otherwise would cause deformation of the dendrimer structure (see Figure 2).

In another embodiment of the invention, a DNA dendrimer as described herein is a two-layer dendrimer. In another embodiment of the invention, a DNA dendrimer as described herein comprises at least 60 biotin molecules. In another embodiment of the invention, a DNA dendrimer as described herein comprises at least 80 biotin molecules. In another embodiment of the invention, a DNA dendrimer as described herein comprises at least 100 biotin molecules. In another embodiment of the invention, a DNA dendrimer as described herein comprises at least 120 biotin molecules. In another embodiment of the invention, a DNA dendrimer as described herein comprises at least 150 biotin molecules. In another embodiment of the invention, a DNA dendrimer as described herein comprises at least 180 biotin molecules. In another embodiment of the invention, a DNA dendrimer as described herein comprises at least 200 biotin molecules. In another embodiment of the invention, a DNA dendrimer as described herein comprises from 1 to 400 biotin molecules. In another embodiment of the invention, a DNA dendrimer as described herein comprises from 60 to 360 biotin molecules. In another embodiment of the invention, a DNA dendrimer as described herein comprises from 80 to 300 biotin molecules. In another embodiment of the invention, a DNA dendrimer as described herein comprises from 100 to 250 biotin molecules. In another embodiment of the invention, a DNA dendrimer as described herein comprises from 150 to 250 biotin molecules. In another embodiment of the invention, a DNA dendrimer as described herein comprises ~120 biotin molecules.

In another embodiment of the invention, a DNA dendrimer as described herein is a 4-layer dendrimer. In another embodiment of the invention, a DNA dendrimer as described herein comprises at least 500 biotin molecules. In another embodiment of the invention, a DNA dendrimer as described herein comprises at least 550 biotin molecules. In another embodiment of the invention, a DNA dendrimer as described herein comprises at least 600 biotin molecules. In another embodiment of the invention, a DNA dendrimer as described herein comprises at least 650 biotin molecules. In another embodiment of the invention, a DNA dendrimer as described herein comprises at least 700 biotin molecules. In another embodiment of the invention, a DNA dendrimer as described herein comprises from 500 to 1500 biotin molecules. In another embodiment of the invention, a DNA dendrimer as described herein comprises from 550 to 1400 biotin molecules. In another embodiment of the invention, a DNA dendrimer as described herein comprises from 600 to 1000 biotin molecules. In another embodiment of the invention, a DNA dendrimer as described herein comprises from 600 to 800 biotin molecules. In another embodiment of the invention, a DNA dendrimer as described herein comprises from 700 to 800 biotin molecules. In another embodiment of the invention, a DNA dendrimer as described herein comprises ~720 biotin molecules.

In another embodiment of the invention, a DNA dendrimer as described herein comprises a complementary capture oligonucleotide ligated to the 5' ends of available arms via a T4 DNA ligase dependent ligation reaction. In another embodiment of the invention, a DNA dendrimer as described herein comprises a complementary capture oligonucleotide (5-80 bases) ligated to the 5' ends of available arms via a T4 DNA ligase dependent ligation reaction. In another embodiment of the invention, a DNA dendrimer as described herein comprises a complementary capture oligonucleotided (10-60 bases) ligated to the 5' ends of available arms via a T4 DNA ligase dependent ligation reaction. In another embodiment of the invention, a DNA dendrimer as described herein comprises a complementary capture oligonucleotide (20-40 bases) ligated to the 5' ends of available arms via a T4 DNA ligase dependent ligation reaction. In another embodiment of the invention, a DNA dendrimer as described herein comprises a complementary capture oligonucleotide (30-50 bases) ligated to the 5' ends of available arms via a T4 DNA ligase dependent ligation reaction. In another embodiment of the invention, a DNA dendrimer as described herein comprises a complementary capture oligonucleotide (30-40 bases) ligated to the 5' ends of available arms via a T4 DNA ligase dependent ligation reaction.

In another embodiment of the invention, a DNA dendrimer as described herein comprises an antibody or a fragment thereof bound to the DNA dendrimers by first covalently conjugating a DNA oligonucleotide (complement to Cap03 sequence) to the antibody using cross-linking condensation conjugation chemistry, followed by hybridization of the antibody-bound oligonucleotide to a complementary sequence (Cap03) on the arms of the dendrimer. In another embodiment of the invention, sequences other than Cap03 can be used for attaching a molecule such as an antibody to the DNA dendrimer.

In another embodiment of the invention, a DNA dendrimer as described herein comprises a siRNA molecule. The siRNA molecule may be chemically synthesized. The siRNA molecule may comprises a single stranded "sense" strand containing a 5 prime portion as RNA ribonucleotides, from 10-25 bases long, and an 3 prime portion as DNA deoxyribonucleotides, typically 0-40 bases long, which is designed to be complementary to the capture oligo ligated to the DNA dendrimer. The siRNA molecule may comprises a single stranded "sense" strand containing a 5 prime portion as RNA ribonucleotides, from 1-30 bases long, and an 3 prime portion as DNA deoxyribonucleotides, typically 1-50 bases long, which is designed to be complementary to the capture oligo ligated to the DNA dendrimer.

In another aspect a siRNA as described herein comprises a single stranded "antisense" strand complementary to the "sense" strand, containing a portion of RNA ribonucleotides only and 5-40 bases long and 1-5 3-prime terminal deoxynucleotides. In another aspect, a siRNA as described herein comprises a single stranded "antisense" strand complementary to the "sense" strand, containing a portion of RNA ribonucleotides only and 10-25 bases long and 1-3 3-prime terminal deoxynucleotides. In another aspect, the invention, the siRNA constructs comprises a 24-35 base extension. In another aspect, a siRNA as described herein comprises two strands combined in equimolar quantities to form stable hybrids between the "sense" and "antisense" strands, leaving the single stranded DNA portion of the "sense" strand available for hybridization to the DNA dendrimer's capture oligonucleotide.

A composition comprising DNA dendrimer as described herein may be used for *in-vitro* transfection. A composition comprising DNA dendrimer as described herein may be used for *in-vivo* transfection. A composition comprising DNA dendrimer and a siRNA molecule as described herein may be used for de-novo knockdown of a transcribed target gene. A siRNA molecule may be designed to target a transcribed target gene (mRNA).

Further described herein are methods for the manufacture and use of a DNA dendrimer containing a targeting antibody or a fragment thereof and a siRNA molecule where the siRNA molecules are non-covalently bound via the binding of biotinylated siRNA molecules to streptavidin, with subsequent binding to biotin on a DNA dendrimer. Also described are methods for the manufacture and use of a DNA dendrimer containing a targeting antibody or a fragment thereof and a siRNA molecule where the siRNA molecules are non-covalently bound via the binding of biotinylated siRNA molecules to streptavidin, with subsequent binding to biotins on a DNA dendrimer.

A composition as described herein comprising a DNA dendrimer bound with targeting antibody or a fragment thereof or any other molecule as provided may be prepared as described above, except that biotin moieties are introduced onto the "arms" of the dendrimers through the hybridization and cross-linking of DNA or RNA oligonucleotides containing end labeled or internal biotins incorporated during the synthesis of the oligos. In another aspect, a typical dendrimer biotin labeling reaction occurs prior to the binding of the antibody or a fragment thereof to the dendrimer, and during or after the ligation of the capture sequence.

In another aspect, a biotinylated "sense" RNA forms an extremely strong non-covalent bond with 2-3 of the 4 available biotin binding valences available on the streptavidin molecule, leaving at least one free biotin binding streptavidin valence (on average) capable of binding a biotin moiety otherwise not associated with the "sense" RNA molecule.

Further described herein are methods for the manufacture of a DNA dendrimer comprising a targeting antibody or a fragment thereof and a siRNA molecule where the siRNA molecules are covalently bound via the use of disulfide bridging bonds. In another aspect, a four layer DNA dendrimer or a two layer DNA dendrimer with antibody or a fragment thereof is synthesized according to in Example 1 or 2. In another aspect, molecules designed to perform as siRNAs within the cell are chemically synthesized and comprise 1) a single stranded "sense" strand comprising all RNA ribonucleotides, typically 10-40 bases long, with 2-80 DNA nucleotides on the 3' end, and containing a sulhydryl (SH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another aspect, these two strands are combined in equimolar quantities to form stable hybrids between the "sense" and "antisense" strands, leaving the sulfhydryl moiety available for conjugation to another sulfhydryl moiety (to form a "S-S" disulfide bond) on a DNA oligonucleotide complementary to a capture sequence attached to the arms of the dendrimer.

Further described herein are methods for the manufacture of a DNA dendrimer containing a targeting antibody or a fragment thereof and a siRNA molecule where the siRNA molecules are covalently bound via the use of NHS-ester dependent condensation chemistry. In another aspect, a four layer DNA dendrimer or a two layer DNA dendrimer with antibody or a fragment thereof is synthesized as in Example 2 except that the biotins are replaced with primary amines. In another aspect, molecules designed to perform as siRNAs within the cell are chemically synthesized and comprise 1) a single stranded "sense" strand comprising all RNA ribonucleotides, typically 5-50 bases long, with 2-80 DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another aspect, the RNA strand containing the carboxyl is chemically modified using commercially available reagents such that the carboxyl was converted to an *N-*hydroxysuccinimide (NHS) ester, which in turn was reacted with a primary amine to form a covalent bond between the NHS ester and the amine. In another aspect, a dendrimer labeled with primary amines is covalently bound with the "sense" strand of the siRNA, which when hybridized with the "antisense" RNA strand forms a functional siRNA duplex.

Further described herein are methods for the manufacture of a DNA dendrimer containing a targeting antibody or a fragment thereof and a siRNA molecule where the siRNA molecules are covalently bound via the use of heterobifunctional chemical cross-linker chemistry. In another aspect, four layer DNA dendrimer or two layer DNA dendrimer with antibody or a fragment thereof is synthesized as in Example 2, except that the biotin molecules were replaced with primary amines. In another aspect, molecules designed to perform as siRNAs within the cell were chemically synthesized and comprise 1) a single stranded "sense" strand comprising all RNA ribonucleotides, typically 5-50 bases long, with 2-80 DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and 5-50 bases long, with two 3' terminal deoxynucleotides. In another aspect, the RNA strand containing the carboxyl is combined with the amine modified dendrimer in the presence of EDC (1-ethyl-3-[3-dimethylaminopropyl]carbodiimide), a heterobifunctional cross-linking reagent that formed a covalent bond between the carboxyl and amine moieties. In another aspect, a dendrimer labeled with primary amines is covalently bound with the "sense" strand of the siRNA, which when hybridized with the "antisense" RNA strand formed a functional siRNA duplex.

Further described herein are methods for the manufacture of a DNA dendrimer containing a targeting antibody or a fragment thereof and a siRNA molecule where the siRNA molecules are covalently bound via the use of a homobifunctional chemical cross-linker chemistry. In another aspect, four layer DNA dendrimer or two layer DNA dendrimer with antibody or a fragment thereof is synthesized as in Example 2, except that the biotins are replaced with primary amines. In another aspect, molecules designed to perform as siRNAs within the cell are chemically synthesized and comprised 1) a single stranded "sense" strand comprising all RNA ribonucleotides, 5-40 bases long, with 2-80 DNA nucleotides on the 3' end, and containing a primary amine moiety attached during or after oligonucleotide synthesis on the 5' end of the molecule, and 2) a single stranded "antisense" strand complementary to the "sense" strand, containing a portion of RNA ribonucleotides only and 2-50 bases long, with two 3' terminal deoxynucleotides. In another aspect, the RNA strand containing the amine is combined with the amine modified dendrimer in the presence of a homobifunctional cross-linker such as Sulfo-EGS [ethylene glycolbis(succinimidylsuccinate)], a reagent that forms a covalent bond between the amine moieties. In another aspect, a DNA dendrimer labeled with primary amines is covalently bound with the "sense" strand of the siRNA, which was then hybridized with the "antisense" RNA strand to form a functional siRNA duplex.

Further described herein are methods for the manufacture of a DNA dendrimer containing a targeting antibody or a fragment thereof and a siRNA molecule and comparing the importance of biotin bound to the structure of the dendrimer as well as cations having multiple positive charges as counterions in transfection. In another aspect, a two layer DNA dendrimer or a four layer DNA dendrimer with antibody or a fragment thereof is synthesized with up to 160 biotins populating the "arms" of the dendrimer, and a certain number of free "arms" on the dendrimer are available for binding of siRNA duplexes via a hybridization binding event (see Example 1 and 2). In another aspect, a two layer DNA dendrimer or a four layer DNS dendrimer with antibody or a fragment thereof is synthesized with up to 140 biotins populating the "arms" of the dendrimer, and a certain number of free "arms" on the dendrimer are available for binding of siRNA duplexes via a hybridization binding event (see Example 1 and 2). In another aspect, a two layer DNA dendrimer or a four layer DNA dendrimer with antibody or a fragment thereof is synthesized with up to 120 biotins populating the "arms" of the dendrimer, and a certain number of free "arms" on the dendrimer are available for binding of siRNA duplexes via a hybridization binding event (see Example 1 and 2).

A composition as described herein may comprise a siRNA construct comprising 10-40 base extension of the sense strand of the siRNA duplex and ICAM1 targeted dendrimers similar to those in Examples 1 and 2. A composition comprising biotinylated dendrimer constructs and a siRNA molecule may be more efficient for transfection than compositions lacking a biotin, especially in the presence of serum. A composition comprising a cation may be more efficient for transfection than compositions lacking a cation, especially in the presence of serum. A composition comprising about 25mM cation may be more efficient for transfection than compositions lacking a cation, especially in the presence of serum. A composition comprising about 5-1000mM cation may be more efficient for transfection than compositions lacking a cation, especially in the presence of serum. A composition may further comprises about 25mM of Mg, Ca, spermine, spermidine, or Mn and may be more efficient for transfection than compositions lacking a cation, especially in the presence of serum. A composition may further comprise about 5-1000mM of Mg, Ca, spermine, spermidine, or Mn and may be more efficient for transfection than compositions lacking a cation, especially in the presence of serum.

Further described herein, are methods for knockdown of mRNA expression utilizing the compositions as described herein. A composition comprising a protective group such as an antibody or a fragment thereof on both the two layer and four layer versions may be synthesized with up to 180 biotins populating the "arms" of the two layer dendrimer, and up to 800 biotins populating the arms of the four layer dendrimer, with both types of dendrimers containing a certain number of free "arms" available for binding of siRNA duplexes via a hybridization binding event (see Examples 1 and 7).

Further described herein are methods for protecting a composition against nuclease dependent degradation of DNA dendrimers from exposure to protein nucleases in human and animal sera. Also described herein is a method for protecting a composition of the invention from protein DNases degradation. Also described herein is a method for protecting a composition of the invention from exogenous DNase degradation.

Further described herein are methods for combining a composition comprising a DNA dendrimer having hybridized siRNA molecules with commercial Lipofectamine transfection reagents. Compositions comprising DNA dendrimers may be combined with a transfection reagent for the cytoplasmic delivery of siRNA. A composition is described comprising a DNA dendrimer and Lipofectamine which improves the knockdown efficiency of the composition. A composition comprising a DNA dendrimer and a liposomal transfection agent is described which has an improved mRNA knockdown efficiency of siRNA molecules. A composition is described comprising a DNA dendrimer and other transfection agents familiar to one skilled in the art which has an improved mRNA knockdown efficiency of siRNA molecules.

The compositions as described herein may be provided to an individual *per se.* The compositions as described herein may be used as part of a diagnostic method. The compositions as described herein may be used as part of a therapeutic method. The compositions as described herein may be provided to the individual as part of a pharmaceutical composition where it is mixed with a pharmaceutically acceptable carrier.

A "pharmaceutical composition" refers to a preparation of one or more of the active ingredients described herein with other chemical components such as physiologically suitable carriers and excipients. The purpose of a pharmaceutical composition is to facilitate administration of a compound to an organism.

Active ingredient" refers to the compositions as described herein, which are accountable for the biological effect.

Described herein are also combined combined preparations. A combined preparation" defines especially a "kit of parts" in the sense that the combination partners as defined above can be dosed independently or by use of different fixed combinations with distinguished amounts of the combination partners i.e., simultaneously, concurrently, separately or sequentially. The parts of the kit of parts can then, e.g., be administered simultaneously or chronologically staggered, that is at different time points and with equal or different time intervals for any part of the kit of parts. The ratio of the total amounts of the combination partners can be administered in the combined preparation. The combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

The phrases "physiologically acceptable carrier" and "pharmaceutically acceptable carrier", which may be interchangeably used, refer to a carrier or a diluent that does not cause significant irritation to an organism and does not abrogate the biological activity and properties of the administered compound. An adjuvant is included under these phrases. One of the ingredients included in the pharmaceutically acceptable carrier can be for example polyethylene glycol (PEG), a biocompatible polymer with a wide range of solubility in both organic and aqueous media (Mutter et al., 1979).

"Excipient" refers to an inert substance added to a pharmaceutical composition to further facilitate administration of an active ingredient. Excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

Suitable routes of administration, for example, include oral, rectal, transmucosal, transnasal, intestinal or parenteral delivery, including intramuscular, subcutaneous and intramedullary injections as well as intrathecal, direct intraventricular, intravenous, inrtaperitoneal, intranasal, or intraocular injections.

The preparation may be administered in a local rather than systemic manner, for example, via injection of the preparation directly into a specific region of a patient's body.

Various embodiments of dosage ranges are described herein. The dosage of the composition described herein is in the range of 0.005-80 mg/day. The dosage may be in the range of 0.05-50 mg/day. The dosage may be in the range of 0.1-20 mg/day. The dosage may be in the range of 0.1-10 mg/day. The dosage may be in the range of 0.1-5 mg/day. The dosage may be in the range of 0.5-5 mg/day. The dosage may be in the range of 0.5-50 mg/day. The dosage may be in the range of 5-80 mg/day. The dosage may be in the range of 35-65 mg/day. The dosage may be in the range of 35-65 mg/day. The dosage may be in the range of 20-60 mg/day. The dosage may be in the range of 40-60 mg/day. The dosage may be in a range of 45-60 mg/day. The dosage may be in the range of 40-60 mg/day. The dosage may be in a range of 60-120 mg/day. The dosage may be in the range of 120-240 mg/day. The dosage may be in the range of 40-60 mg/day. The dosage may be in the range of 40-60 mg/day. The dosage may be in a range of 240-400 mg/day. The dosage may be in a range of 400-800 mg/day. The dosage may be in a range of 800-1600 mg/day. The dosage may be in a range of 45-60 mg/day. The dosage may be in the range of 15-25 mg/day. The dosage may be in the range of 5-10 mg/day. The dosage may be in the range of 55-65 mg/day.

The dosage may be 20 mg/day. The dosage may be 30 mg/day. The dosage may be 40 mg/day. The dosage may be 50 mg/day. The dosage may be 60 mg/day. The dosage may be 70 mg/day. The dosage may be 80 mg/day. The dosage may be 90 mg/day. The dosage may be 100 mg/day.

Peroral compositions may comprise liquid solutions, emulsions, suspensions, and the like. Pharmaceutically-accebtable carriers suitable for preparation of such compositions are well known in the art. Liquid oral compositions may comprise from about 0.012% to about 0.933% of the desired compound or compounds, or from about 0.033% to about 0.7%. The oral dosage form may comprise a predefined release profile.

Compositions for use in the methods may comprise solutions or emulsions, which may be aqueous solutions or emulsions comprising a safe and effective amount of the compounds of the present invention and optionally, other compounds, intended for topical intranasal administration. Compositions may comprise from about 0.01% to about 10.0% w/v of a subject compound, more preferably from about 0.1% to about 2.0, which is used for systemic delivery of the compounds by the intranasal route.

The pharmaceutical compositions may be administered by intravenous, intra-arterial, or intramuscular injection of a liquid preparation. Liquid formulations may include solutions, suspensions, dispersions, emulsions, oils and the like. The pharmaceutical compositions may be administered intravenously, and are thus formulated in a form suitable for intravenous administration. The pharmaceutical compositions may be administered intra-arterially, and thus formulated in a form suitable for intra-arterial administration. The pharmaceutical compositions may be administered intramuscularly, and thus may be formulated in a form suitable for intramuscular administration.

The pharmaceutical compositions may be administered topically to body surfaces, and thus may be formulated in a form suitable for topical administration. Suitable topical formulations include gels, ointments, creams, lotions, drops and the like. For topical administration, the compounds of the present invention are combined with an additional appropriate therapeutic agent or agents, prepared and applied as solutions, suspensions, or emulsions in a physiologically acceptable diluent with or without a pharmaceutical carrier.

Pharmaceutical compositions may be manufactured by processes well known in the art, e.g., by means of conventional mixing, dissolving, granulating, dragee-making, levigating, emulsifying, encapsulating, entrapping or lyophilizing processes.

Pharmaceutical compositions may be formulated in conventional manner using one or more physiologically acceptable carriers comprising excipients and auxiliaries, which facilitate processing of the active ingredients into preparations which, can be used pharmaceutically. Formulation is dependent upon the route of administration chosen.

Injectables may be formulated in aqueous solutions. Injectables may be formulated in physiologically compatible buffers such as Hank's solution, Ringer's solution, or physiological salt buffer. For transmucosal administration, penetrants appropriate to the barrier to be permeated may be used in the formulation. Such penetrants are generally known in the art.

The preparations described herein may be formulated for parenteral administration, e.g., by bolus injection or continuous infusion. Formulations for injection may be presented in unit dosage form, e.g., in ampoules or in multidose containers with optionally, an added preservative. Compositions may be suspensions, solutions or emulsions in oily or aqueous vehicles, and contain formulatory agents such as suspending, stabilizing and/or dispersing agents.

The compositions may also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise, local anesthetics or other actives. The compositions can be used as sprays, mists, drops, and the like.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acid esters such as ethyl oleate, triglycerides or liposomes. Aqueous injection suspensions may contain substances which increase the viscosity of the suspension, such as sodium carboxymethyl cellulose, sorbitol or dextran. The suspension may also contain suitable stabilizers or agents which increase the solubility of the active ingredients to allow for the preparation of highly concentrated solutions.

The active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

The pharmaceutical composition delivered in a controlled release system may be formulated for intravenous infusion, implantable osmotic pump, transdermal patch, liposomes, or other modes of administration. A pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). Polymeric materials can be used. A controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

The active ingredient may be in powder form for constitution with a suitable vehicle, e.g., sterile, pyrogen-free water based solution, before use. Compositions may be formulated for atomization and inhalation administration. Compositions may be contained in a container with attached atomizing means.

Pharmaceutical compositions suitable for use include compositions wherein the active ingredients are contained in an amount effective to achieve the intended purpose. A therapeutically effective amount means an amount of active ingredients effective to prevent, alleviate or ameliorate symptoms of disease or prolong the survival of the subject being treated.

Determination of a therapeutically effective amount is well within the capability of those skilled in the art.

The compositions also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment of the invention, the pharmaceutically-acceptable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. Peroral liquid compositions may also contain one or more components such as sweeteners, flavoring agents and colorants disclosed above.

The compositions also include incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of in vivo release, and rate of in vivo clearance.

Also included are particulate compositions coated with polymers (e.g. poloxamers or poloxamines) and the compound coupled to antibodies directed against tissue-specific receptors, ligands or antigens or coupled to ligands of tissue-specific receptors.

Compounds may be modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. The modified compounds may exhibit substantially longer half-lives in blood following intravenous injection than do the corresponding unmodified compounds. Modifications may also increase the compound's solubility in aqueous solution, eliminate aggregation, enhance the physical and chemical stability of the compound, and greatly reduce the immunogenicity and reactivity of the compound. The desired in vivo biological activity may be achieved by the administration of such polymer-compound abducts less frequently or in lower doses than with the unmodified compound.

Preparation of effective amount or dose can be estimated initially from in vitro assays. A dose can be formulated in animal models and such information can be used to more accurately determine useful doses in humans.

Toxicity and therapeutic efficacy of the active ingredients-compositions as described herein can be determined by standard pharmaceutical procedures in vitro, in cell cultures or experimental animals. In one embodiment of the invention, the data obtained from these in vitro and cell culture assays and animal studies can be used in formulating a range of dosage for use in human. The dosages vary depending upon the dosage form employed and the route of administration utilized. The exact formulation, route of administration and dosage can be chosen by the individual physician in view of the patient's condition. [See e.g., Fingl, et al., (1975) "The Pharmacological Basis of Therapeutics", Ch. 1 p.1]

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions including the preparation of the present invention formulated in a compatible pharmaceutical carrier may also be prepared, placed in an appropriate container, and labeled for treatment of an indicated condition.

Compositions may be presented in a pack or dispenser device, such as an FDA approved kit, which contain one or more unit dosage forms containing the active ingredient. The pack, for example, may comprise metal or plastic foil, such as a blister pack. The pack or dispenser device may be accompanied by instructions for administration. The pack or dispenser may be accommodated by a notice associated with the container in a form prescribed by a governmental agency regulating the manufacture, use or sale of pharmaceuticals, which notice is reflective of approval by the agency of the form of the compositions or human or veterinary administration. Such notice, may be labeling approved by the U.S. Food and Drug Administration for prescription drugs or of an approved product insert.

The composition can be provided to the individual with additional active agents to achieve an improved therapeutic effect as compared to treatment with each agent by itself. Measures (e.g., dosing and selection of the complementary agent) are taken to adverse side effects which are associated with combination therapies.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document.

### EXAMPLE 1

### Manufacture and use of a DNA dendrimer containing a targeting antibody or a fragment thereof and siRNA molecules non-covalently bound via a hybridization event

### MATERIALS AND METHODS:

DNA dendrimers are constructed from DNA monomers, each of which was made from two DNA strands that share a region of sequence complementarity located in the central portion of each strand. When the two strands anneal to form the monomer the resulting structure can be described as having a central double-stranded "waist" bordered by four single-stranded "arms". This waist-plus-arms structure comprises the basic DNA monomer. The single-stranded arms at the ends of each of the five monomer types are designed to interact with one another in precise and specific ways. Base-pairing between the arms of complementary monomers allowed directed assembly of the dendrimer through sequential addition of monomer layers (Figure 1). Assembly of each layer of the dendrimer included a cross-linking process where the strands of DNA were covalently bonded to each other, thereby forming a completely covalent molecule impervious to denaturing conditions that otherwise would cause deformation of the dendrimer structure (Figure 2). Further as described in Example 2 the dendrimers prepared for this example contained ~120 biotin molecules (2-layer dendrimers) or ~720 biotin molecules (4-layer dendrimers) attached to their outer surface. In addition, 38 base oligonucleotides that serve as complementary capture oligos were ligated to the 5' ends of available dendrimer arms via a simple T4 DNA ligase dependent ligation reaction, as follows:

**Table 1: The components that were added to a microfuge tube**

| | |
|---|---|
| two layer DNA dendrimer (500ng/uL) in 1X TE buffer | 5.4uL (2680ng) |
| a(-)LIG-BR7 Bridging oligo (14mer) (50ng/uL) | 2.7uL (134ng) |
| 10X Ligase buffer | 10.2uL |
| Nuclease free water | 81.7uL |
| Cap03 capture oligo (38mer) (50ng/uL) | 4.0uL (200ng) |
| T4 DNA Ligase (1 U/uL) | 10.0uL (10 units) |

The first four reactants were added together, heated to 65°C and cooled to room temperature. The 5^{th} and 6^{th} reactants were then added and incubated for 45 minutes. The ligation reaction was stopped by adding 2.8uL of 0.5M EDTA solution. Non-ligated oligonucleotide was removed via the use of a size exclusion spin column prepared using Sephacryl S400 (Pharmacia).

Antibodies were bound to DNA dendrimers by first covalently conjugating a DNA oligonucleotide (Complement to Cap03 sequence) to the antibody using previously described cross-linking condensation conjugation chemistry (prepared at Solulink Inc. (San Diego, CA), followed by hybridization of the antibody-bound oligonucleotide to a complementary sequence (Cap03) on the arms of the dendrimer. This hybridization comprises 31 base pairs and has a melting temperature of greater than 65°C. in a physiological salt solution, thereby providing a stable complex of dendrimer bound with antibody at physiological temperatures and conditions. A typical hybridization formulation inluded:

**Table 2: components that were added to a microfuge tube:**

| | |
|---|---|
| two layer DNA dendrimer with ligated Cap03 sequence (50ng/uL) | 50.0uL |
| 50% ethelyene glycol in PBS or equivalent (e.g. Superfreeze (Pierce)) | 25.0uL |
| 1X Phosphate Buffered Saline (PBS) | 57.0uL |
| 5M NaCl | 4.3uL |
| Oligo-Antibody Conjugate (anti-mouse ICAM-1 antibody) (7.8ng/uL as oligo) | 13.7uL |

The above reactants were combined, gently mixed and incubated at 37°C for 30 minutes. This formulation is stable at 4°C for at least six months.

### SiRNA Design/Preparation

Molecules designed to perform as siRNAs within the cell were chemically synthesized and comprise 1) a single stranded "sense" strand containing a 5 prime portion as RNA ribonucleotides, typically 19 bases long, and an 3 prime portion as DNA deoxyribonucleotides, typically 0-33 bases long, which is designed to be complementary to the capture oligo ligated to the DNA dendrimer, and 2) a single stranded "antisense" strand complementary to the "sense" strand, containing a portion of RNA ribonucleotides only and typically 19 bases long and two 3 prime terminal deoxynucleotides. These two strands are combined in equimolar quantities to form stable hybrids between the "sense" and "antisense" strands, leaving the single stranded DNA portion of the "sense" strand available for hybridization to the DNA dendrimer's capture oligonucleotide.

For this experiment, the length of the deoxynucelotide extension (that links the siRNA to the dendrimer) versus the effectiveness of the siRNA when attached to 3 DNA molecules was studied. ICAM-1 on Hepa 1-6 cells was targeted and the Hepa 1-6 cells were grown in Delbecco's Modified Eagles Media (DMEM) with and without 10% fetal bovine serum (FBS) and used siRNAs designed to knockdown mouse ssb (La autoantigen) (Catalog number: S101433831, Qiagen GMBH, Hilden, Germany) mRNA verses a siRNA having no target (Catalog number: S1027310, Qiagen GMBH, Hilden,Germany).

SiRNAs Tested:

### Condition #1: No Linker

### SSB SiRNAs:

### Antisense strand:

RNA antisense strand : 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand: 5'- rUrUrA rArArG rUrCrU rGrUrU rGrUrC rArGrC rC dGdG -3' (SEQ ID NO: 10)

r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'-dTdT-3'

The complete sense strand is composed of the DNA sense strand bound to the 3' end of the RNA sense strand thus forming a RNA-DNA hybrid sense strand:
5'-rGrGrC rUrGrA rCrArA rCrArG rArCrU rUrUrA rA dTdT-3' (SEQ ID NO: 11)
r-refers to a ribonucleotide; d- refers to a deoxyribonucleotide;

### Negative Control siRNA:

### Antisense strand:

RNA antisense strand: 5'- ACGUGACACGUUCGGAGAA-3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUC UCCGAACGUGUCACGU -3' (SEQ ID NO: 4)
DNA sense strand: 5'-dTdT-3'

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT -3' (SEQ ID NO: 13)
r-refers to a ribonucleotide; d- refers to a deoxyribonucleotide;

### Condition #2: SSB with 16 base DNA linker sequence:

### Antisense strand:

RNA antisense strand: 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'- TTCCGTTGACATCTCGTA -3' (SEQ ID NO: 5)

The complete sense strand is composed of the DNA sense strand bound to the 3' end of the RNA sense strand thus forming a RNA-DNA hybrid sense strand: r-refers to a ribonucleotide; d- refers to a deoxyribonucleotide;

### Negative Control (no mRNA target) with 16 base linker sequence:

### Antisense strand:

RNA antisense strand: 5'-ACGUGACACGUUCGGAGAA-3' (SEQ ID NO: 3)
DNA antisense strand 5'-dGdG-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT-3' (SEQ ID NO: 17).
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU-3' (SEQ ID NO: 4)
DNA sense strand: 5'- TTCCGTTGACATCTCGTA -3' (SEQ ID NO: 5)

The complete sense strand is composed of the DNA sense strand bound to the 3' end of the RNA sense strand thus forming a RNA-DNA hybrid sense strand: r-refers to a ribonucleotide; d- refers to a deoxyribonucleotide;

### Condition #3: SSB with 21 base DNA linker sequence:

### Antisense strand:

RNA antisense strand: 5'-UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

Sense strand:
RNA sense strand: 5'-GGCUGACAACAGACUUUAA-3' (SEQ ID NO: 2)
DNA sense strand: 5'-TTCCGTTGACATCTCGTAGATTT-3' (SEQ ID NO: 6)

The complete sense strand is composed of the DNA sense strand bound to the 3' end of the RNA sense strand thus forming a RNA-DNA hybrid sense strand:

### Negative Control (no mRNA target) with 21 base linker sequence:

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU -3' (SEQ ID NO: 4)
DNA sense strand: 5'- TTCCGTTGACATCTCGTAGATTT -3' (SEQ ID NO: 6)

The complete sense strand is composed of the DNA sense strand bound to the 3' end of the RNA sense strand thus forming a RNA-DNA hybrid sense strand: r-refers to a ribonucleotide; d- refers to a deoxyribonucleotide;

### Condition #4: SSB with 26 base DNA linker sequence:

### Antisense strand:

RNA antisense strand: 5'- UUAAAGUCUGUUGUCAGCC -3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- GGCUGACAACAGACUUUAA-3' (SEQ ID NO: 2)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand: r-refers to a ribonucleotide; d- refers to a deoxyribonucleotide;

### Negative Control (no mRNA target) with 26 base linker sequence:

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU -3' (SEQ ID NO: 4)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT dCdCdGdTdT dGdAdC dAdTdCdTdC dGdTdAdGdAdTdTdTdG dAdAdTdT -3' (SEQ ID NO: 15)
r-refers to a ribonucleotide; d- refers to a deoxyribonucleotide;

### SiRNA hybridization formulation:

"Sense" DNA/RNA molecule (50 microMolar) 25 µL
"Antisense" RNA molecule (50 microMolar) 25 µL

The sense and antisense siRNA oligonucleotides were combined in a microfuge per the formulation above. The oligo mixture was incubated at 80°C for 5 minutes then transferred to 37°C for 20 minutes to form the siRNA duplex. Prior to the preparation of a transfection mixture the hybridized siRNA was diluted by a factor of 25 fold in serum free media to a final concentration of 2 microMolar.

### Preparation of Transfection Mixtures

The following components were combined in a microfuge tube:
two layer DNA dendrimer with Antibody (10ng/µL) 12.0 µL
"SiRNA Duplex molecule (diluted to 2 microMolar) 3.0 µL
"Serum Free Media or PBS 105.0 µL

This mixture was incubated at 37°C for 20-30 minutes and then place at room temperature until use or at 4°C for longer term storage. For experiments in which other agents were added to the transfection mixture, the volume of the added component was subtracted from the amount of serum free media used in the transfection mixture. For example, transfection mixtures containing MgCl₂ were prepared by adding 7.5µl of 1M MgCl₂ and 97.5µl of serum free media in place of the 105µl of serum free media.

### Transfection Experiments:

The DNA dendrimer, containing the targeting antibody and the hybridized siRNA duplex, was introduced into wells in a tissue culture plate containing 2,000-10,000 live cells suitable as targets for *in-vitro* transfection and grown in the appropriate media containing 10% serum or in serum free media. These cells must contain certain features, including but not limited to 1) surface antigens suitable as binding targets for the dendrimer bound targeting antibody, 2) messenger RNA (mRNA) that will serve as an appropriate target for the siRNA antisense molecule bound to the dendrimer, 3) the ability to internalize the DNA dendrimer via an antibody mediated cell surface binding event, or other event(s) capable of initiating an endocytosis process. Typically, the above formulation was added to 100µL of tissue culture media in a 96 well plate at a volume ranging from 10-25% of the total volume of the well (10-25µl), although less or more may be required for the best effect. Function of the siRNA was measured directly by quantifying the amount of intact mRNA remaining in the cell after the addition of the dendrimer-siRNA complex using a qRT-PCR assay designed to detect and the targeted mRNA relative to an internal control mRNA (18s RNA and PPIB mRNA), or was measured indirectly by quantifying the amount of a protein remaining in the cell after the transfection of the siRNA and knockdown of expression of the mRNA target and associated protein(s).

As a control to confirm appropriate function of modified siRNAs, knockdown activity was confirmed for each modified siRNA using a commercially available transfection reagent, Lipofectamine 2000 (Invitrogen, Carlsbad, CA) according to the manufacture's recommendation and a final siRNA concentration of 10 nanoMolar. All knockdown determinations were relative to a Negative Control (no mRNA target) siRNA duplex containing the same structural modifications.

List of SiRNA modifications:
1. Wild-type siRNA Duplex consisting of both Control Unmodified unextended strands.
2. SiRNA Duplex consisting of the Sense Strand with a 16 base deoxynucleotide extension.
3. SiRNA Duplex consisting of the Sense Strand with a 21 base deoxynucleotide extension.

SiRNA Duplex consisting of the Sense Strand with a 26 base deoxynucleotide extension.

### Results

First the knockdown efficiency was measured for each of the siRNAs (10 nanoMolar final concentration) independent of the DNA dendrimer delivery method using Lipofectamine 2000 by measuring the relative amount of SSB mRNA remaining compared to the appropriate negative control oligo. In all cases we observed between 70-95% knockdown efficiency. We then prepared DNA dendrimer hybridization mixtures having a 10nanoMolar final siRNA concentration and 0.2nanogram per microliter final DNA dendrimer concentration (∼2.5-3 nanoMolar as dendrimer bound siRNA for those capable of binding to dendrimer via the sequence extension) and compared knockdown efficiencies. In general, it was observed more significant knockdown efficiency in serum free media compared to serum containing media, most likely because of degradation of the siRNA. In both serum containing and serum free media comparisons, the siRNA constructs containing the longest (26 base) extension compared to the two shorter 3' deoxynucleotide extensions (21 and 16 bases, and no extension, respectively) performed best, yielding more knockdown of the target mRNA. In serum containing media we observed approximately 40% knockdown for the siRNA dulex with the 26 base 3' extension ranging down to little or no knockdown where the siRNA had no 3' extension of the sense strand. In serum free media, we observed approximately 65-70% knockdown for the 26 base 3' extended siRNA compared to 0-5%, 10-20%, and 30-35% knockdown for no 3' extension, 16 base extension, and 21 base extension, respectively.

Based on the results for the transfection comparing serum containing media to serum free media, the investigations continued to include siRNA modified with various chemical moieties in order to improve the stability of the siRNA in serum containing media. These modifications are listed above in the specification.

### EXAMPLE 2

### Manufacture and use of a DNA dendrimer containing a targeting antibody and siRNA molecules where the siRNA molecules are non-covalently bound via the binding of biotinylated siRNA molecules to streptavidin, with subsequent binding to biotins on a DNA dendrimer

DNA dendrimers, bound with targeting antibodies are prepared as described above, except that biotin moieties are introduced onto the "arms" of the dendrimers through the hybridization and cross-linking of DNA or RNA oligonucleotides containing end labeled biotins incorporated during the synthesis of the oligos. A typical dendrimer biotin labeling reaction occurred prior to the binding of the antibody to the dendrimer, and during or after the ligation of the capture sequence, as follows:

The following components were added to a microfuge tube:
four layer DNA dendrimer with ligated Cap03 sequence (50ng/µL) 50.0µL
c(-) biotin oligo (500ng/µL) 2.6µL
a(-) biotin oligo (500ng/µL) 2.6µL
5M NaCl 4.0µL
2,4,8 trimethyl psoralen saturated in ethanol 7.0µL

The above reactants were added together, mixed well, and placed into a container of water at 65° and slow cooled to 42°C. Exposure to 300nm UV light for 10 minutes (X2) initiated a cross-linking event covalently binding the biotinylated oligos to the arms of the DNA dendrimer. Non-cross-linked oligonucleotides were removed via the use of a size exclusion spin column.

Biotin labeled oligonucleotides were sourced from commercial DNA oligonucleotide vendors. A variety of biotinylated phosphoramidites for synthesis of the biotinylated DNA oligonucleotides were used, including DNA synthesis reagents available from Glen Research Inc.and Trilink Biotechnology Inc., and include but are not limited to Biotin Phosphoramidite (Glen Research Cat # 10-1953-95), BiotinTEG Phosphoramidite (Glen Research Cat # 10-1955-95), Biotin-dT (Glen Research Cat # 10-1038-95), 5'-Biotin Phosphoramidite (Glen Research Cat # 10-5950-95), 5' Biotin (Trilink), Biotin Diol Linker (5' or Internal) (Trilink), 3' Biotin BB CPG (Trilink), and 5' Dual Biotin (Trilink). Other methods for the incorporation of biotin into nucleic acids using enzymatic and chemical synthesis will likely result in similar labeling efficiencies, including the incorporation of biotin into DNA using DNA polymerases and biotinylated deoxyribonucleotides, the incorporation of biotin into RNA using RNA polymerases and biotinylated ribonucleotides, and the chemical incorporation of biotin into nucleic acids using technologies commercially available from Kreatech, Mirus Bio and other companies.

Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a biotin moiety (or biotin analog) attached during or after oligonucleotide synthesis on the 3' or 5' end of the molecule, and 2) a single stranded "antisense" strand complementary to the "sense" strand, containing a portion of RNA ribonucleotides only and typically 19 bases long, with two 3' terminal deoxyribonucleotides. These two strands were combined in equimolar quantities to form stable hybrids between the "sense" and "antisense" strands, leaving the biotin moiety available for binding to an avidin or streptavidin molecule. The biotin-avidin binding formulation was:
SiRNA hybridization formulation:
   "Sense" DNA/RNA molecule (50 microMolar) with end biotin label 25 µL
   "Antisense" RNA molecule (50 microMolar) 25 µL

The sense and antisense siRNA oligonucleotides were combined in a microfuge per the formulation above. The oligo mixture was incubated at 80°C for 5 minutes then transferred to 37°C for 20 minutes to form the siRNA duplex.

The following components were added to a microfuge tube:
Hybridized duplex siRNA with end biotin label (50uM) 5.3µL
1X PBS 86.5µL
Streptavidin (1000ng/µL) 8.0µL
5M NaCl 0.2µL

The above reactants were combined, gently mixed and incubated at 37°C for 10 minutes.

In the above formulation, the biotinylated "sense" RNA formed an extremely strong non-covalent bond with 2-3 of the 4 available biotin binding valences available on the streptavidin molecule, leaving at least one free biotin binding streptavidin valence (on average) capable of binding a biotin moiety otherwise not associated with the "sense" RNA molecule.

The [biotinylated siRNA-streptavidin] complex was then mixed with the biotinylated dendrimer such that the remaining biotin binding valence(s) on the streptavidin bound to the biotin labels on the DNA dendrimer. This binding was accomplished by the following reaction:
four layer biotinylted DNA dendrimer with Antibody (10ng/µL) 50.0µL
"biotinylated siRNA-streptavidin" complex 5.3µL
1X PBS 3.7µL

The above reactants were combined, gently mixed and incubated at 37°C for 30 minutes.

The DNA dendrimer, containing the targeting antibody and the hybridized siRNA duplex, was introduced into wells in a tissue culture plate containing 2,000-10,000 live cells suitable as targets for *in-vitro* transfection. These cells contained certain features, including but not limited to 1) surface antigens suitable as binding targets for the dendrimer bound targeting antibody, 2) messenger RNA (mRNA) that served as an appropriate target for the siRNA antisense molecule bound to the dendrimer, 3) the ability to internalize the DNA dendrimer via an antibody mediated cell surface binding event, or other event(s) capable of initiating an endocytosis or internalization process. The above formulation was added to 100µL of tissue culture media in a 96 well plate at a volume ranging from 10-25% of the total volume of the well (10-25µl), although less or more may be required for the best effect. Function of the siRNA was measured directly by quantifying the amount of intact mRNA remaining in the cell after the addition of the dendrimer-siRNA complex, or was measured indirectly by quantifying the amount of a protein synthesized by the cell as a result of the degradation activity of the siRNA binding to a specific mRNA, resulting from the "knockdown" of expression of the mRNA and associated protein(s).

### EXAMPLE 3

### Manufacture of a DNA dendrimer containing a targeting antibody and siRNA molecules where the siRNA molecules are covalently bound via the use of disulfide bridging bonds

Four layer DNA dendrimer with Antibody (10ng/µL) is synthesized as in Example 1 or 2 above. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a sulhydryl (SH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. These two strands are combined in equimolar quantities to form stable hybrids between the "sense" and "antisense" strands, leaving the sulfhydryl moiety available for conjugation to another sulfhydryl moiety (to form a "S-S" disulfide bond) on a DNA oligonucleotide complementary to a capture sequence attached to the arms of the dendrimer. A typical sulfhydryl-sulfhydryl conjugation to a disulfide formulation would be:
The following components were added to a microfuge tube:
   "Sense" siRNA strand with end sulfhydryl (50uM) 10.0µL
   DNA oligo complementary to capture sequence with sulfhydryl (50uM) 9.0µL
   2M Dithiothreatol (DTT) aqueous 20.0µL
   Nuclease free water 1.0µL

The above reactants were combined, gently mixed and incubated at 65°C for 16 hours, or until most or all disulfide bonds were reduced to single sulfhydryl moieties. After incubation, the mixture was desalted and the buffer exchanged via the use of a commercial desalting column (Pierce, cat# 89891), yielding an equimolar solution of the "sense" RNA molecule and the DNA molecule, both containing S-H sulfhydryls resulting from the reduction of the disulfide formed after oligonucleotide synthesis. Stable disulfide bonds were formed between the DNA and RNA oligos either in the presence of mild oxidative conditions (exposure to air, oxygen or ozone) or via the addition of a mild oxidizing agent (hydrogen peroxide, 1-3%). On average, approximately 50% of the DNA and RNA disulfide complexes resulting from random formation of the disulfide bond will be of the appropriate DNA/RNA oligo combination, with 25% of the combinations comprising DNA/DNA and RNA/RNA disulfide complexes. The DNA/RNA complexes, which comprised a molecular weight and total length unique from the DNA/DNA and RNA/RNA complexes, were purified on an HPLC or via PAGE electrophoresis processes. The resulting DNA/RNA complex containing the disulfide bond between the DNA and RNA oligos was then hybridized to the DNA dendrimer as discussed in Example 1.

### EXAMPLE 4

### Manufacture of a DNA dendrimer containing a targeting antibody and siRNA molecules where the siRNA molecules are covalently bound via the use of NHS-ester dependent condensation chemistry

Four layer DNA dendrimer with Antibody (10ng/uL) was synthesized as in Example 2 above, except that the biotins were replaced with primary amines. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. Typically, the RNA strand containing the carboxyl was chemically modified using commercially available reagents such that the carboxyl was converted to an *N-*hydroxysuccinimide (NHS) ester, which in turn was reacted with a primary amine to form a covalent bond between the NHS ester and the amine. A dendrimer labeled with primary amines can thus be covalently bound with the "sense" strand of the siRNA, which when hybridized with the "antisense" RNA strand forms a functional siRNA duplex.

The conversion of the carboxyl to the NHS was performed as below:
The following components were added to a microfuge tube:
   "Sense" siRNA strand with end carboxyl (500ng/µL) in ultrapure water 1000µL
   EDC (1-ethyl-3-[3-dimethylaminopropyl]carbodiimide) 0.4mg
   Sulfo-NHS reagent (Pierce cat# 24510) 1.1mg

After incubation for 15 minutes at room temperature (15-30°C), the mixture was desalted and the buffer exchanged via the use of a commercial desalting column (Pierce, cat# 89891). The exchange buffer was 1X PBS pH 7.4 (containing no amines).

Next, the "sense" RNA molecule now containing an activated NHS ester was added to a DNA dendrimer containing primary amines (in water or 1X PBS buffer containing no amines). This reaction was allowed to proceed for 2 hours at room temperature. After the incubation, 1M Tris-HCl was added to a final concentration of 50mM, which "quenched" the reaction of the NHS-esters. 5M NaCl was added to a final concentration of 100mM. The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column as previously described.

### EXAMPLE 5

### Manufacture of a DNA dendrimer containing a targeting antibody and siRNA molecules where the siRNA molecules are covalently bound via the use of a heterobifunctional chemical cross-linker chemistry

Four layer DNA dendrimer with Antibody (10ng/µL) was synthesized as in Example 2 above, except that the biotin molecules were replaced with primary amines. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a carboxyl (COOH) moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. The RNA strand containing the carboxyl was combined with the amine modified dendrimer in the presence of EDC (1-ethyl-3-[3-dimethylaminopropyl]carbodiimide), a heterobifunctional cross-linking reagent that formed a covalent bond between the carboxyl and amine moieties. Thus, a dendrimer labeled with primary amines was covalently bound with the "sense" strand of the siRNA, which when hybridized with the "antisense" RNA strand formed a functional siRNA duplex.

The crosslinking of the "sense" RNA strand containing a carboxyl to the primary amines pre-bound to the dendrimer was performed as below:
The following components were added to a microfuge tube:
   "Sense" siRNA strand with end carboxyl (500ng/µL) in ultrapure water 100.0µL
   four layer amine dendrimer with capture sequence (500ng/µL) in 1X PBS 100.0µL
   EDC (1-ethyl-3-[3-dimethylaminopropyl]carbodiimide) 10.0mg

The above reaction was incubated for 2 hours at room temperature (15-30°C). After incubation, the mixture was desalted and the buffer exchanged via the use of a commercial desalting column (Pierce, cat# 89891). The exchange buffer used was 1X PBS pH 7.4 (containing no amines).

The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column. Antibody was also bound to the dendrimer as described in Examples 1 and 2.

### EXAMPLE 6

### Manufacture of a DNA dendrimer containing a targeting antibody and siRNA molecules where the siRNA molecules are covalently bound via the use of a homobifunctional chemical cross-linker chemistry

Four layer DNA dendrimer with Antibody (10ng/µL) was synthesized as in Example 2 above, except that the biotins are replaced with primary amines. Molecules designed to perform as siRNAs within the cell were chemically synthesized and comprised 1) a single stranded "sense" strand comprising all RNA ribonucleotides, typically 19-23 bases long, with two or more DNA nucleotides on the 3' end, and containing a primary amine moiety attached during or after oligonucleotide synthesis on the 5' end of the molecule, and 2) a single stranded "antisense" strand complementary to the "sense" strand, containing a portion of RNA ribonucleotides only and typically 19 bases long, with two 3' terminal deoxynucleotides. The RNA strand containing the amine was combined with the amine modified dendrimer in the presence of a homobifunctional cross-linker such as Sulfo-EGS [ethylene glycolbis(succinimidylsuccinate)] (Pierce cat# 21566), a reagent that forms a covalent bond between the amine moieties. Thus, a dendrimer labeled with primary amines was covalently bound with the "sense" strand of the siRNA, which was then hybridized with the "antisense" RNA strand to form a functional siRNA duplex.

The crosslinking of the "sense" RNA strand containing a primary amine to the primary amines pre-bound to the dendrimer was performed as below:
The following components were added to a microfuge tube:
   "Sense" siRNA strand with end amine (500ng/µL) in ultrapure water 100.0µL
   four layer amine dendrimer with capture sequence (500ng/µL) in 1X PBS 100.0µL
   Sulfo-EGS [ethylene glycolbis(succinimidylsuccinate)] 20.0mg

The above reaction was incubated for 2 hours at room temperature (15-30°C). After incubation, the mixture was desalted and the buffer exchanged via the use of a commercial desalting column (Pierce, cat# 89891). The exchange buffer used was 1X PBS pH 7.4 (containing no amines).

The "antisense" RNA strand was added in excess and allowed to hybridize with dendrimer bound "sense" RNA strand by warming the reaction to 37°C for 30 minutes. Unreacted or excess reagents were removed from the dendrimer via the use of a size exclusion spin column. Antibody was also bound to the dendrimer as described in Examples 1 and 2.

### EXAMPLE 7

### Manufacture of a DNA dendrimer containing a targeting antibody and siRNA molecules and comparing the importance of biotin bound to the structure of the dendrimer as well as cations having multiple positive charges as counterions in transfection

A two layer DNA dendrimer with antibody was synthesized with up to 120 biotins populating the "arms" of the dendrimer, and a certain number of free "arms" on the dendrimer are available for binding of siRNA duplexes via a hybridization binding event (see Example 1 and 2).

Using a siRNA construct consisting of a 26 base extension of the sense strand of the siRNA duplex (best result in Example 1) and ICAM1 targeted dendrimers similar to those in Examples 1 and 2, we examined the importance of biotin on the dendrimer delivery platform. For this experiment we compared dendrimers prepared with and without biotin on the outer surface. The same methods for dendrimer and a siRNA molecule preparation as well as combining of the two and used in transfection knockdown studies were used as described in Example 1. Further in combination with biotinylated dendrimer constructs in siRNA knockdown assays we compared targeted dendrimer siRNA transfection efficiency with or without Mg 2+, Ca 2+, and Mn2+ in the assay as part of the initial hybridization of siRNA dendrimer construct by adding 1M of the appropriate cation salt solution to a final concentration of 125 mM and subsequently using 25ul of this mixture combined with plated cells in 100µl of serum or serum free media.

### Results

We observed that dendrimer constructs prepared with biotin on the outer surface demonstrated a 65-80% knockdown, while dendrimers without biotin only produced approximately 35-40% knockdown when tested in serum free media (as described in Example 1). In serum containing media a similar ratio of performance was observed between dendrimer with biotin verses without biotin, the siRNA knockdown activity being about 25-50% of that observed in serum free media for dendrimers without biotin compared to their biotinylated counterpart. Similarly, we observed that Mg, Ca, and Mn at a final concentration of 25mM yielded more reproducible and efficient knockdown compared to transfections in which cation was omitted. We also expect that other cations such as spermine and spermidine will demonstrate similar benefits when using dendrimers in these types of experiments.

### EXAMPLE 8

### Use of 2 and four layer dendrimers for siRNA knockdown of mRNA expression

DNA dendrimers with antibody on both the two layer and four layer versions are synthesized with up to 120 biotins populating the "arms" of the two layer dendrimer, and up to 720 biotins populating the arms of the four layer dendrimer, with both types of dendrimers containing a certain number of free "arms" available for binding of siRNA duplexes via a hybridization binding event (see Examples 1 and 7). The same methods for siRNA preparation, combining of dendrimer with siRNA, and transfection knockdown studies were used as described in Example 1. 2-layer versus 4-layer dendrimer results were compared. The final siRNA and dendrimer concentration (by mass) were used for both 2 and four layer constructs to insure that equal amounts of siRNA molecules were bound to dendrimer molecules.

### Results

The results indicate when using equal input mass of each dendrimer type at the same siRNA final concentration that 2-layer dendrimers produce 1.5-2 fold greater knockdown than do 4-layer dendrimer even though each 2-layer dendrimer has 1/9^{th} the amount of siRNA molecules per dendrimer.

### EXAMPLE 9

### Protection from nuclease dependent degradation of DNA dendrimers from exposure to protein nucleases in human and animal sera

Unmodified DNA dendrimers are subject to nuclease dependent degradation when exposed to solutions containing protein DNases. Therefore, it was logical to presume that DNA dendrimers, either unmodified or modified with various hapten, flourescent, amine or other labels and targeting antibodies, would quickly degrade when introduced into an *in-vitro* or *in-vivo* environment containing fluids derived from animal sources (e.g serum). This assumption historically precluded our use of DNA dendrimers for *in-vitro* cellular assays and any *in-vivo* applications until very recently, when it was unexpectedly observed that DNA dendrimers (prepared according to the prior examples 1-8) showed significant resistance to nuclease dependent degradation for at least 960 minutes (16 hours). The experiment was performed as follows:

Experiment 1: incubation of unmodified and modified DNA dendrimer with 0% and 75% human serum, with and without additional exogenous DNase added.

### Conditions:

Tubes 1-4 contained unmodified four layer DNA dendrimers, with the following additives:
Tube 1: In PBS only.
Tube 2: In 75% fresh human serum.
Tube 3: In PBS with 1U of exogenous DNase.
Tube 4: In 75% fresh human serum with 1U of exogenous DNase.

Tubes 5-8 contained modified four layer DNA dendrimers, containing ∼960 FITC dyes per dendrimer (on average) and 15-25 anti-ICAM1 mouse monoclonal antibodies (attached as described in example 1), with the following additives:
Tube 5: In PBS only.
Tube 6: In 75% fresh human serum.
Tube 7: In PBS with 1U of exogenous DNase.
Tube 8: In 75% fresh human serum with 1U of exogenous DNase.

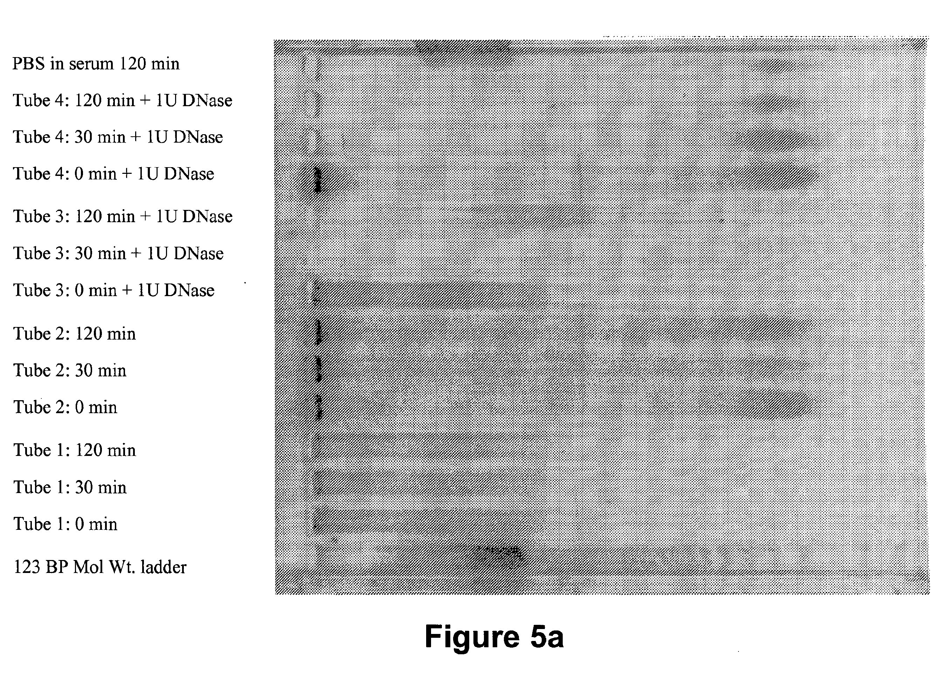

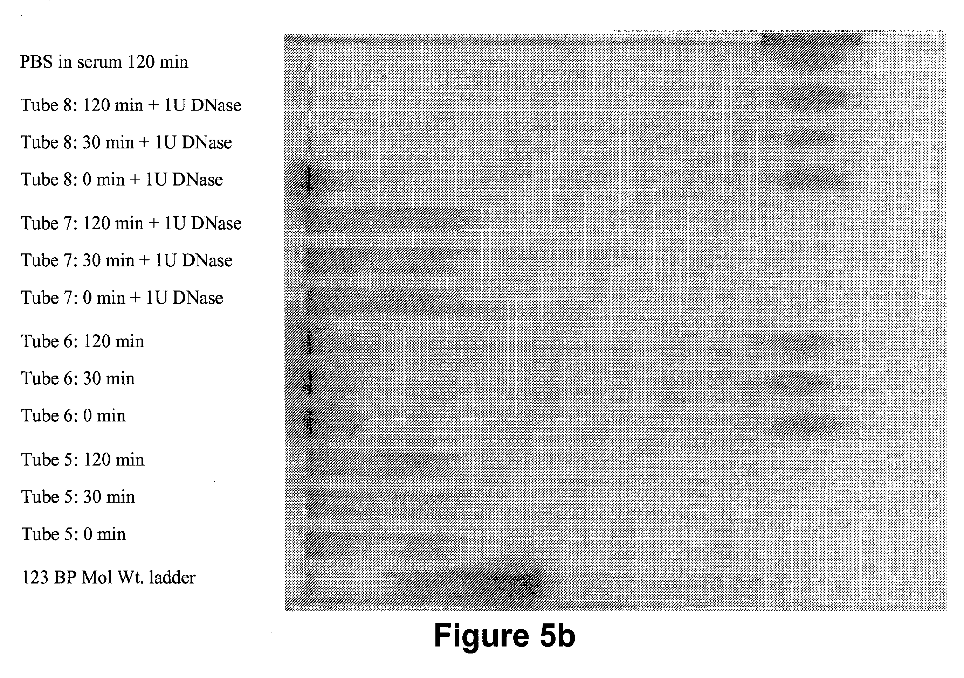

Each tube was incubated at 37°C for 0, 30 and 120 minutes. Samples were removed from each tube at each time-point and EDTA was added immediately to the removed samples to a final concentration of 50mM in order to stop the nuclease degradation via simple chelation of critical cations required for nuclease activation. Degradation of the dendrimers was observed after each samples was electrophoresed for 3 hours on an 0.8% agarose gel at 75 volts (figures 5a and 5b).

The gel results clearly indicated the following results:
Tube 1: No degradation observed after 120 minutes.
Tube 2: No degradation observed after 120 minutes.
Tube 3: Obvious degradation observed at the 30 minute timepoint.
Tube 4: Obvious degradation observed at the 30 minute timepoint.
Tube 5: No degradation observed after 120 minutes.
Tube 6: No degradation observed after 120 minutes.
Tube 7: No degradation observed after 120 minutes.
Tube 8: Obvious degradation observed at the 30 minute timepoint.

### Results

Degradation of unmodified DNA dendrimers occurred only in the presence of 1U of exogenous DNase in both the PBS and 75% serum conditions. Degradation of the modified DNA dendrimers occurred only in the presence of 1U of exogenous DNase in the 75% serum condition only.

*Conclusion:* both the unmodified and modified DNA dendrimers demonstrated unexpected resistance to nuclease degradation in human serum, although the modified DNA dendrimer further showed resistance to exogenous DNase in the PBS buffer but not in the human serum. This was an unexpected result, as prior results indicated that unmodified DNA dendrimers were severely degraded after a relatively brief exposure (30 minutes) to animal serum.

Experiment 2: incubation of unmodified and modified DNA dendrimer with 0% and 75% human serum for intervals up to 960 minutes (16 hours).

### Conditions

Tubes 1-4 contained unmodified four layer DNA dendrimers, with the following additives:
Tube 1: PBS only.
Tube 2: 75% fresh human serum.

Tubes 3-4 contained modified four layer DNA dendrimers, containing ∼960 FITC dyes per dendrimer (on average) and 15-25 anti-ICAM1 mouse monoclonal antibodies (attached as described in example 1), with the following additives:
Tube 3: PBS only.
Tube 4: 75% fresh human serum.

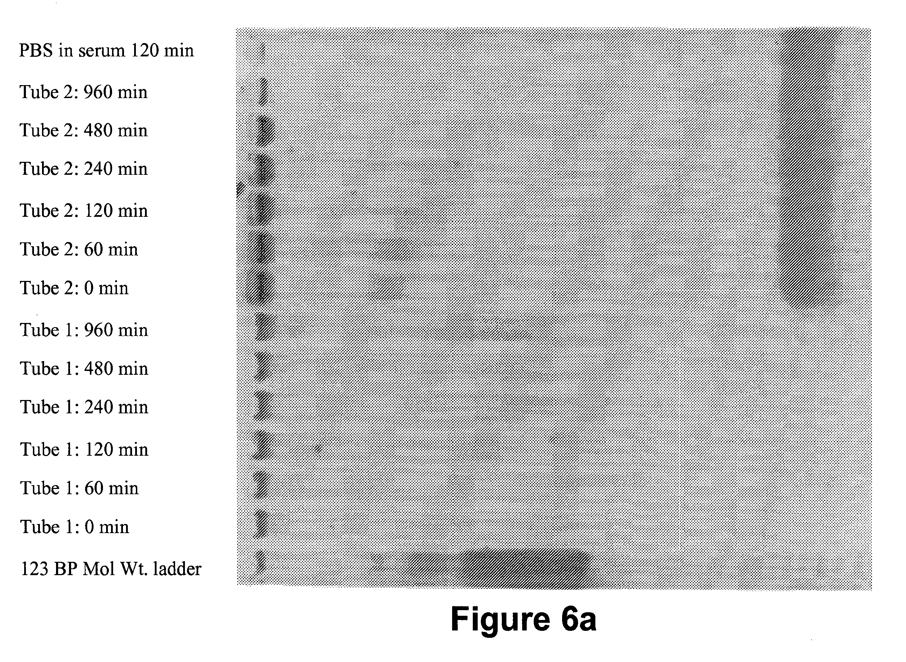

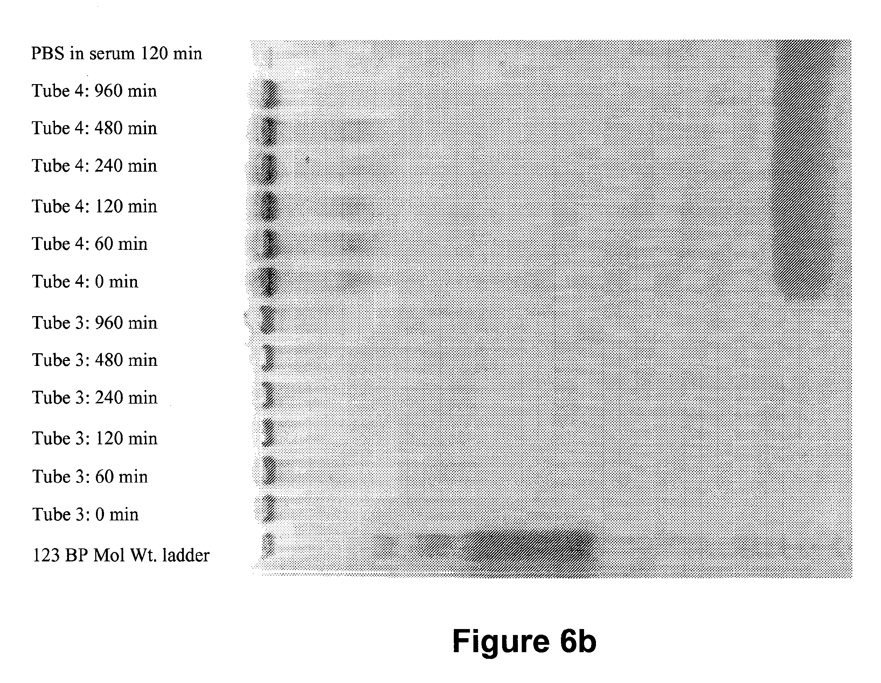

Each tube was incubated at 37°C for 0, 60 and 120, 240, 480 and 960 minutes. Samples were removed from each tube at each time-point and 50mM EDTA was added immediately to the removed samples to stop the nuclease degradation via simple chelation of critical cations required for nuclease activation. Degradation of the dendrimers was observed after each samples was electrophoresed for 3 hours on an 0.8% agarose gel at 75 volts (figures 6a and 6b The gel results clearly indicated the following results:
Tube 1: No degradation observed after 960 minutes.
Tube 2: No degradation observed after 480 minutes, with >80% degradation observed at the 960 minute time point.
Tube 3: No degradation observed after 960 minutes.
Tube 4: No degradation observed after 960 minutes.

### Results

Degradation of unmodified DNA dendrimers occurred only in the presence of 75% human serum sometime after the 480 minute time-point. Degradation of the modified DNA dendrimers was not observed at any timepoint for either the PBS or 75% serum conditions.

*Conclusion:* both the unmodified and modified DNA dendrimers demonstrated unexpected resistance to nuclease degradation in human serum, although the modified DNA dendrimer further showed somewhat more resistance to serum based nuclease degradation when compared to the unmodified DNA dendrimer. This was also an unexpected result, demonstrating the stability of modified DNA dendrimers for at 16 hours in 75% fresh human serum at 37°C. We believe that the addition of the FITC and antibody modifications to the DNA dendrimer provides some additional level of protection otherwise not expected from prior experiences with DNA molecules otherwise not chemically modified to resist nucleases in human serum (e.g. phosphothiorate chemistry modification, which was not used for these DNA dendrimers).

### EXAMPLE 10

### Combination of DNA dendrimers having hybridized siRNA molecules with commercial Lipofectamine transfection reagents

The goal of this experiment was to determine if DNA dendrimers independent of a targeting antibody and having a siRNA molecule attached via hybridization can be successfully combined with another transfection reagent for the cytoplasmic delivery of siRNA as measured by mRNA knockdown.

DNA dendrimers were prepared as outlined in example 1 and as previously disclosed (see patents 5,175,270, 5,484,904, 5,487,973, 6,110,687, and 6,274,723). Briefly, a DNA dendrimer was constructed from DNA monomers, each of which was made from two DNA strands that share a region of sequence complementarity located in the central portion of each strand. When the two strands anneal to form the monomer the resulting structure can be described as having a central double-stranded "waist" bordered by four single-stranded "arms". This waist-plus-arms structure comprises the basic DNA monomer. The single-stranded arms at the ends of each of the five monomer types are designed to interact with one another in precise and specific ways. Base-pairing between the arms of complementary monomers allowed directed assembly of the dendrimer through sequential addition of monomer layers (figure 1). Assembly of each layer of the dendrimer included a cross-linking process where the strands of DNA were covalently bonded to each other, thereby forming a completely covalent molecule impervious to denaturing conditions that otherwise would cause deformation of the dendrimer structure (figure 2). The dendrimers prepared for this example contained either no biotin or up to ∼720 biotin molecules (4-layer dendrimers) attached to their outer surface. For some conditions tested antibodies were combined with dendrimers by first attaching 38 base oligonucleotides that serve as complementary capture oligos were ligated to the 5' ends of available dendrimer arms via a simple T4 DNA ligase dependent ligation reaction, as follows:

The following components were added to a microfuge tube:
two layer DNA dendrimer (500ng/µL) in 1X TE buffer 5.4µL (2680ng)
a(-)LIG-BR7 Bridging oligo (14mer) (50ng/µL) 2.7µL (134ng)
10X Ligase buffer 10.2µL
Nuclease free water 81.7µL
Cap03 capture oligo (38mer) (50ng/µL) 4.0µL (200ng)
T4 DNA Ligase (1 U/µL) 10.0µL (10 units)

The first four reactants were added together, heated to 65°C and cooled to room temperature. The 5^{th} and 6^{th} reactants were then added and incubated for 45 minutes. The ligation reaction was stopped by adding 2.8µL of 0.5M EDTA solution. Non-ligated oligonucleotide was removed via the use of a size exclusion spin column prepared using Sephacryl S400 (Pharmacia).

Antibodies were bound to DNA dendrimers by first covalently conjugating a DNA oligonucleotide (Complement to Cap03 sequence) to the antibody using previously described cross-linking condensation conjugation chemistry (prepared at Solulink Inc. (San Diego, CA), followed by hybridization of the antibody-bound oligonucleotide to a complementary sequence (Cap03) on the arms of the dendrimer. This hybridization comprises 31 base pairs and has a melting temperature of greater than 65°C. in a physiological salt solution, thereby providing a stable complex of dendrimer bound with antibody at physiological temperatures and conditions.

A typical hybridization formulation:
two layer DNA dendrimer with ligated Cap03 sequence (50ng/µL) 50.0µL
50% ethelyene glycol in PBS or equivalent (e.g. Superfreeze (Pierce)) 25.0µL
1X Phosphate Buffered Saline (PBS) 57.0µL
5M NaCl 4.3µL
Oligo-Antibody Conjugate (anti-mouse ICAM-1 antibody) (7.8ng/µL as oligo) 13.7µL

The above reactants were combined, gently mixed and incubated at 37°C for 30 minutes. This formulation is stable at 4°C for at least six months.

### SiRNA Design/Preparation

Molecules designed to perform as siRNAs within the cell are chemically synthesized and comprise 1) a single stranded "sense" strand containing a 5 prime portion as RNA ribonucleotides, typically 19 bases long, and an 3 prime portion as DNA deoxyribonucleotides, typically 0-33 bases long, which is designed to be complementary to the capture oligo ligated to the DNA dendrimer, and 2) a single stranded "antisense" strand complementary to the "sense" strand, containing a portion of RNA ribonucleotides only and typically 19 bases long and two 3 prime terminal deoxynucleotides. These two strands are combined in equimolar quantities to form stable hybrids between the "sense" and "antisense" strands, leaving the single stranded DNA portion of the "sense" strand available for hybridization to the DNA dendrimer's capture oligonucleotide.

For this experiment we studied siRNA molecules either directly attached to dendrimers via hybridization of a 26 base long linker sequence or unattached. In some cases, antibodies were pre-attached to dendrimer as outlined by the conditions listed below.

For antibody containing dendrimers we attached anti-ICAM-1 because this antibody is observed on the cell surface of Hepa 1-6 cells grown in Delbecco's Modified Eagles Media (DMEM) with and without 10% fetal bovine serum (FBS). For all cases we used siRNAs designed to knockdown mouse ssb (La autoantigen) mRNA verses a siRNA having no target (Qiagen).

### SiRNAs Tested:

### siRNAs without dendrimer attachment sequence:

### SSB SiRNAs (SSB unmod):

### Antisense strand:

RNA antisense strand: 5'- UUAAAGUCUGUUGUCAGCC-3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'-GGCUGACAACAGACUUUAA-3' (SEQ ID NO: 2)
DNA sense strand: 5'-dTdT-3'

The complete sense strand is composed of the DNA sense strand bound to the 3' end of the RNA sense strand thus forming a RNA-DNA hybrid sense strand:
5'-rGrGrC rUrGrA rCrArA rCrArG rArCrU rUrUrA rA dTdT-3' (SEQ ID NO: 11)
r-refers to a ribonucleotide; d- refers to a deoxyribonucleotide;

### Negative Control SiRNA (Neg unmod):

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUC UCCGAACGUGUCACGU -3' (SEQ ID NO: 4)
DNA sense strand: 5'-dTdT-3'

The complete sense strand is composed of the DNA sense strand bound to the 3' end of the RNA sense strand thus forming a RNA-DNA hybrid sense strand:
5'- rUrUrC rUrCrC rGrArA rCrGrU rGrUrC rArCrG rU dTdT -3' (SEQ ID NO: 13).
r-refers to a ribonucleotide; d- refers to a deoxyribonucleotide;

### SSB with 26 base DNA linker sequence (SSB+26):

### Antisense strand:

RNA antisense strand: 5'- UUAAAGUCUGUUGUCAGCC -3' (SEQ ID NO: 1)
DNA antisense strand 5'-dGdG-3'

The complete antisense strand is composed of the DNA antisense strand bound to the 3' end of the RNA antisense strand thus forming a RNA-DNA hybrid antisense strand:
5'- rUrUrA rArArG rUrCrU rGrUrU rGrUrC rArGrC rC dGdG -3' (SEQ ID NO: 10)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- GGCUGACAACAGACUUUAA-3' (SEQ ID NO: 2)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand:
5'- rGrGrC rUrGrA rCrArA rCrArG rArCrU rUrUrA rA dTdT dCdCdGdTdT dGdAdC dAdTdCdTdC dGdTdAdGdAdTdTdTdG dAdAdTdT -3' (SEQ ID NO: 14)
r-refers to a ribonucleotide; d- refers to a deoxyribonucleotide;

### Negative Control (no mRNA target) with 26 base linker sequence:

### Antisense strand:

RNA antisense strand: 5'- ACGUGACACGUUCGGAGAA -3' (SEQ ID NO: 3)
DNA antisense strand 5'-dTdT-3'

The complete antisense strand is composed of the DNA antisense strand bound to the 3' end of the RNA antisense strand thus forming a RNA-DNA hybrid antisense strand:
5'- rArCrG rUrGrA rCrArC rGrUrU rCrGrG rArGrA rA dTdT -3' (SEQ ID NO: 12)
r-refers to a ribonucleotide; d- refers to a deoxyribonucleotide; underlined nucleotide indicate a position within the oligonucleotide that would have a 2'- methoxy (also referred to as Omethyl) modification.

### Sense strand:

RNA sense strand: 5'- UUCUCCGAACGUGUCACGU -3' (SEQ ID NO: 4)
DNA sense strand: 5'- TTCCGTTGACATCTCGTAGATTTGAATT -3' (SEQ ID NO: 7)

The complete sense strand is composed of the DNA sense strand bound to the 3' end of the RNA sense strand thus forming a RNA-DNA hybrid sense strand: r-refers to a ribonucleotide; d- refers to a deoxyribonucleotide;
SiRNA hybridization formulation:
"Sense" DNA/RNA molecule (50 microMolar) 25 µL
"Antisense" RNA molecule (50 microMolar) 25 µL

The sense and antisense siRNA oligonucleotides were combined in a microfuge tube per the formulation above. The oligo mixture was incubated at 80°C for 5 minutes then transferred to 37°C for 20 minutes to form the siRNA duplex. Prior to the preparation of a transfection mixture the hybridized siRNA was diluted by a factor of 25 fold in serum free media to a final concentration of 2 microMolar.

### Preparation of Transfection Mixtures

Lipofectamine 2000 (Invitrogen, Carlsbad, CA) was used according to the manufacturer and first diluted to a 2 times concentrate in serum free media to prepare a 2x Lipofectamine solution.

SiRNA only Lipofectamine complexes:
The following components were combined in a microfuge tube:
   "SiRNA Duplex molecule (diluted to 2 microMolar) 3.0 µL
   "Serum Free Media or PBS 51.0 µL

To the above mixture 54µl of 2x Lipofectamine solution was added 5 minutes prior to use.

Dendrimer plus siRNA Lipofectamine Complexes:
The following components were combined in a microfuge tube:
   four layer DNA dendrimer with or without Antibody (10ng/µL) 12.0 µL
   "SiRNA Duplex molecule (diluted to 2 microMolar) 3.0 µL
   "Serum Free Media or PBS 39.0 µL

This mixture was incubated at 37°C for 20-30 minutes and then place at room temperature until being combined with 2x Lipofectamine solution 5 minutes prior to use.

### Transfection Experiments

Transfection mixtures were introduced into wells in a tissue culture plate containing 2,000-10,000 live cells suitable as targets for *in-vitro* transfection and grown in the appropriate media containing 10% serum or in serum free media. Five microliters of the appropriate above formulation was added to 120uL of tissue culture media in a 96 well plate containing the plated cells. The final concentration of the siRNA was 2 nanomolar. Function of the siRNA was measured directly by quantifying the amount of intact target mRNA remaining in the cell after the addition of the Lipofectamine siRNA or Lipofectamine dendrimer-siRNA complex using a qRT-PCR assay designed to detect and the targeted mRNA (ssb) relative to an internal control mRNA (18s RNA and PPIB mRNA).

All knockdown determinations were relative to a Negative Control (no mRNA target) siRNA duplex containing the same structural modifications.

### List of experimental conditions tested:

Lipofectamine plus "SSB unmod" no dendrimer (#1), Lipofectamine plus "Neg unmod" no dendrimer (#2), Lipofectamine plus "SSB+26" no dendrimer (#3), Lipofectamine plus "Neg+26" no dendrimer (#4), Lipofectamine plus "Dendrimer no Ab plus "SSB unmod" (#5), Lipofectamine plus "Dendrimer no Ab plus "Neg unmod" (#6), Lipofectamine plus "Dendrimer no Ab plus "SSB+26" (#7), Lipofectamine plus "Dendrimer no Ab plus "Neg+26" (#8), Lipofectamine plus "720 biotin Dendrimer no Ab plus "SSB unmod" (#9), Lipofectamine plus "720 biotin Dendrimer no Ab plus "Neg unmod" (#10),Lipofectamine plus "720 biotin Dendrimer no Ab plus "SSB+26" (#11), Lipofectamine plus "720 biotin Dendrimer no Ab plus "Neg+26" (#12), Lipofectamine plus "720 biotin Dendrimer with Ab plus "SSB unmod" (#13), Lipofectamine plus "720 biotin Dendrimer with Ab plus "Neg unmod" (#14), Lipofectamine plus "720 biotin Dendrimer with Ab plus "SSB+26" (#15), Lipofectamine plus "720 biotin Dendrimer with Ab plus "Neg+26" (#16).

### Results

In all cases comparing the knockdown efficiency of "SSB unmod" to "SSB+26" regardless of whether the siRNA was combined with a dendrimer, little difference was observed between the two siRNA constructs, indicating that the 26 base extension was not impacting the results in either a positive or negative manor. Further, when the siRNA was attached to the dendrimer it performed as well as the unhybridized siRNA suggesting that the hybridized siRNA was efficiently released form the dendrimer siRNA construct.

Comparing knockdown efficiency of Lipofectamine siRNA complexes not containing dendrimer (conditions #1-#4 above) to those containing dendrimer (either #5-#8, #9-#12, or #13-#16) we observed a significant improvement in the efficiency of knockdown when dendrimer was present. Lipofectamine complexes with out dendrimer demonstrated about 80% knockdown compared to greater than 90-95% percent knockdown when dendrimers were present.

Little or no difference was observed comparing knockdown efficiency of Lipofectamine siRNA dendrimer complexes with or without antibody. Both were equally efficient.

While dendrimers containing biotin demonstrated a trend of better knockdown efficiency when combined with siRNA in Lipofectamine complexes compared to similar complexes prepared with dendrimers without biotin, no statistical difference of knockdown efficiency was observed at this dose of siRNA.

*Conclusion:* Based on the observed results it was concluded that DNA dendrimers when combined with liposomal transfection agents improve the mRNA knockdown efficiency of siRNA molecules. Based on the compositions used we theorize that dendrimers may operate to improve the release of the siRNA from subcellular compartments (e.g. endosomes).

As set forth above, the foregoing discussion discloses and describes various exemplary and preferred embodiments of the present invention. However, one skilled in the art will readily recognize from such discussion, and from the accompanying drawings, claims, and examples, that changes, modifications, and variations can be made therein.

By way of example, the following non-claimed aspects are mentioned.
1. A composition comprising a DNA dendrimer attached to a siRNA molecule.
2. The composition of aspect 1, wherein a a protein, a molecule containing a polyethylene glycol (PEG) moiety, biotin or a biotin derivative, fluorescein or a fluorescein derivative, or any combination thereof is further attached to said DNA dendrimer.
3. The composition of aspect 2, comprising a DNA dendrimer attached to a siRNA molecule and an antibody or an antibody fragment
4. The composition of aspect 2, comprising a DNA dendrimer attached to a siRNA molecule and biotin.
5. The composition of aspect 2, comprising a DNA dendrimer attached to a siRNA molecule and fluorescein or of fluorescein derivative, including fluorescein isothiocyanate (FITC).
6. The composition of aspect 2, further comprising human or animal serum, plasma, blood, lymph and any number of other bodily fluids.
7. The composition of aspect 2, wherein said composition is stable in serum for at least 0.5 hours.
8. A method of preparing a composition comprising a DNA dendrimer and a siRNA molecule, comprising the step of attaching said siRNA to said DNA dendrimer (a) via a disulfide bridging bond; (b) via the use of NHS ester dependent condensation reaction; (c) via the use of a bifunctional cross linking reaction, (d) via direct or indirect hybridization of the siRNA to DNA dendrimer sequence; or (e) via the use of polycationic compounds to bridge siRNA molecule to said DNA dendrimer via charge-charge interactions.
9. The method of aspect 8, further comprising the step of attaching to said DNA dendrimer a protein, fluorescein or a fluorescein derivative, digoxigenin, cholesterol, a primary amine, a hydrocarbon spacer of length of 3 to 120 carbons, a molecule containing a polyethylene glycol (PEG) moiety, biotin or a biotin derivative, or any combination thereof.
10. The method of aspect 9, further attaching an antibody or a fragment thereof.
11. The method of aspect 9, further attaching biotin.
12. The method of aspect 9, further attaching fluorescein or a fluorescein derivative.
13. A method of protecting a siRNA molecule against degradation in a bodily fluid, comprising the step of attaching said siRNA molecule to a DNA dendrimer and a protein, digoxigenin, cholesterol, a primary amine, a hydrocarbon spacer of length of 3 to 120 carbons, a PEG molecule, biotin, a biotin derivative, fluorescein or a fluorescein derivative, or any combination thereof to a DNA dendrimer, thereby protecting a siRNA molecule against degradation.
14. The method of aspect 13, wherein said degradation is degradation occurring in a bodily fluid.
15. The method of aspect 14, wherein said bodily fluid comprises serum
16. The method of aspect 13, wherein said protein is an antibody or a fragment thereof.
17. The method of aspect 13, comprising the step of attaching said biotin to said DNA dendrimer.
18. The method of aspect 13, comprising the step of attaching said fluorescein or a fluorescein derivative to said DNA dendrimer.
19. A method of protecting a DNA dendrimer against degradation in a bodily fluid, comprising the step of attaching to said DNA dendrimer a protein, digoxigenin, cholesterol, a primary amine, a hydrocarbon spacer of length of 3 to 120 carbons, a molecule containing a PEG moiety, biotin or a biotin derivative, fluorescein or a fluorescein derivative, or any combination thereof, thereby protecting said DNA dendrimer against nuclease degradation in the bodily fluid.
20. The method of aspect 19, wherein said bodily fluid comprises serum.
21. The method of aspect 19, wherein said protein is an antibody or a fragment thereof.
22. The method of aspect 19, comprising the step of attaching said biotin to said DNA dendrimer
23. The method of aspect 19, comprising the step of attaching said fluorescein or a fluorescein derivative to said DNA dendrimer.
24. The method of aspect 19, further comprising the step of attaching to said DNA dendrimer a molecule comprising a DNA molecule, a RNA molecule, a protein, a chemotherapeutic agent, an antiviral agent, an anti-inflammatory agent, a bacteriostatic agent, a psychoactive agent, a statin, a neuropathic agents, a hormone, an ACE inhibitor, an anti-clotting factor, an analgestic, an anti angiogenic agent, a pro angiogenic agent, a growth factor, a growth factor inhibitor, or any combination thereof.
25. A method of delivering a DNA dendrimer into a bodily fluid, comprising the step of attaching to said DNA dendrimer a protein, a molecule containing a PEG moiety, biotin or a biotin derivative, fluorescein or a fluorescein derivative, or any combination, thereby delivering a DNA dendrimer into a bodily fluid.
26. The method of aspect 25, wherein said bodily fluid is serum.
27. The method of aspect 25, wherein said delivering is in- vivo delivering.
28. The method of aspect 25, further comprising the step of attaching to said DNA dendrimer a nucleic acid molecule.
29. The method of aspect 28, wherein said nucleic acid molecule is a siRNA molecule.
30. The method of aspect 25, wherein said protein is an antibody or a fragment thereof.
31. The method of aspect 25, comprising the step of attaching said biotin or a biotin derivative to said DNA dendrimer.
32. The method of aspect 25, comprising the step of attaching said fluorescein or a fluorescein derivative to said DNA dendrimer.

## Claims

1. Use of a compound selected from the group consisting of a protein, digoxigenin, cholesterol, a primary amine, a hydrocarbon spacer of length of 3 to 120 carbons, a PEG molecule, biotin, a biotin derivative, fluorescein or a fluorescein derivative, or any combination thereof, for protecting a DNA dendrimer against nuclease degradation in a bodily fluid, wherein the compound is attached to the dendrimer.

2. The use of claim 1, wherein said compound is an antibody or a fragment thereof.

3. The use of claim 1, wherein said compound is biotin.

4. The use of claim 1, wherein said compound is fluorescein or a fluorescein derivative.

5. The use of any one of the preceding claims, wherein said bodily fluid is a serum.

6. The use of any one of the preceding claims, wherein the DNA dendrimer is further attached to a molecule selected from a group consisting of a siRNA molecule, a RNA molecule and a DNA molecule.

## Patentansprüche

1. Verwendung einer Verbindung, die aus der aus einem Protein, Digoxigenin, Cholesterin, einem primären Amin, einem Kohlenwasserstoff-Spacer mit einer Länge von 3 bis 120 Kohlenstoffatomen, einem PEG-Molekül, Biotin, einem Biotinderivat, Fluorescein oder einem Fluoresceinderivat oder einer beliebigen Kombination davon bestehenden Gruppe ausgewählt ist, zum Schützen eines DNA-Dendrimers gegen Nukleaseabbau in einer Körperflüssigkeit, wobei die Verbindung an das Dendrimer gebunden ist.

2. Verwendung nach Anspruch 1, wobei es sich bei der Verbindung um einen Antikörper oder ein Fragment davon handelt.

3. Verwendung nach Anspruch 1, wobei es sich bei der Verbindung um Biotin handelt.

4. Verwendung nach Anspruch 1, wobei es sich bei der Verbindung um Fluorescein oder ein Fluoresceinderivat handelt.

5. Verwendung nach einem der vorhergehenden Ansprüche, wobei es sich bei der Körperflüssigkeit um ein Serum handelt.

6. Verwendung nach einem der vorhergehenden Ansprüche, wobei das DNA-Dendrimer ferner an ein Molekül gebunden ist, das aus einer aus einem siRNA-Molekül, einem RNA-Molekül und einem DNA-Molekül bestehenden Gruppe ausgewählt ist.

## Revendications

1. Utilisation d'un composé, choisi dans le groupe constitué par une protéine, la digoxigénine, le cholestérol, une amine primaire, un espaceur hydrocarboné d'une longueur de 3 à 120 carbones, une molécule de PEG, la biotine, un dérivé de la biotine, la fluorescéine ou un dérivé de la fluorescéine, ou une quelconque combinaison de ceux-ci, pour la protection d'un dendrimère d'ADN contre une dégradation par nucléase dans un liquide corporel, dans laquelle le composé est fixé au dendrimère.

2. Utilisation selon la revendication 1, dans laquelle ledit composé est un anticorps ou un fragment de celui-ci.

3. Utilisation selon la revendication 1, dans laquelle ledit composé est la biotine.

4. Utilisation selon la revendication 1, dans laquelle ledit composé est la fluorescéine ou un dérivé de la fluorescéine.

5. Utilisation selon l'une quelconque des revendications précédentes, dans laquelle ledit liquide corporel est un sérum.

6. Utilisation selon l'une quelconque des revendications précédentes, dans laquelle le dendrimère d'ADN est fixé en outre à une molécule choisie dans le groupe constitué par une molécule d'ARNsi, une molécule d'ARN et une molécule d'ADN.


### SEQUENCE LISTING

<110> Genisphere, Inc.
<120> LONG-ACTING DNA DENDRIMERS AND METHODS THEREOF
<130> DS10428PCEPT1
<140> Divisional of EP 09 805 654.2
   <141> 2009-08-10
<150> US 61/188,318
   <151> 2008-08-08
<160> 19
<170> PatentIn version 3.5
<210> 1
   <211> 19
   <212> RNA
   <213> Artificial Sequence
<220>
   <223> SSB SiRNA, RNA antisense strand
<400> 1
   uuaaagucug uugucagcc 19
<210> 2
   <211> 19
   <212> RNA
   <213> Artificial Sequence
<220>
   <223> SSB SiRNA, RNA sense strand
<400> 2
   ggcugacaac agacuuuaa 19
<210> 3
   <211> 19
   <212> RNA
   <213> Artificial Sequence
<220>
   <223> Negative Control siRNA, RNA antisense strand
<400> 3
   acgugacacg uucggagaa 19
<210> 4
   <211> 19
   <212> RNA
   <213> Artificial Sequence
<220>
   <223> Negative Control siRNA, RNA sense strand
<400> 4
   uucuccgaac gugucacgu 19
<210> 5
   <211> 18
   <212> DNA
   <213> Artificial Sequence
<220>
   <223> SSB with 16 base DNA linker sequence, DNA sense strand
<400> 5
   ttccgttgac atctcgta 18
<210> 6
   <211> 23
   <212> DNA
   <213> Artificial Sequence
<220>
   <223> SSB with 21 base DNA linker sequence, DNA sense strand
<400> 6
   ttccgttgac atctcgtaga ttt 23
<210> 7
   <211> 28
   <212> DNA
   <213> Artificial Sequence
<220>
   <223> SSB with 26 base DNA linker sequence, DNA sense strand
<400> 7
   ttccgttgac atctcgtaga tttgaatt 28
<210> 8
   <211> 31
   <212> DNA
   <213> Artificial Sequence
<220>
   <223> Cap03 abiotic sequence for structural "capture sequences" attached to DNA dendrimer
<400> 8
   tccaccttag agtacaaacg gaacacgaga a 31
<210> 9
   <211> 31
   <212> DNA
   <213> Artificial Sequence
<220>
   <223> Cap03 abiotic anti-sense sequence attached to DNA dendrimer
<400> 9
   ttctcgtgtt ccgtttgtac tctaaggtgg a 31
<210> 10
   <211> 21
   <212> DNA
   <213> Artificial Sequence
<220>
   <223> siRNAs Antisense strand without dendrimer attachment sequence
<400> 10
   uuaaagucug uugucagccg g 21
<210> 11
   <211> 21
   <212> DNA
   <213> Artificial Sequence
<220>
   <223> siRNAs sense strand without dendrimer attachment sequence
<400> 11
   ggcugacaac agacuuuaat t 21
<210> 12
   <211> 21
   <212> DNA
   <213> Artificial Sequence
<220>
   <223> Negative Control antisense SiRNA
<400> 12
   acgugacacg uucggagaat t 21
<210> 13
   <211> 21
   <212> DNA
   <213> Artificial Sequence
<220>
   <223> Negative Control sense strand SiRNA
<400> 13
   uucuccgaac gugucacgut t 21
<210> 14
   <211> 47
   <212> DNA
   <213> Artificial Sequence
<220>
   <223> Negative Control (no mRNA target) with 26 base linker sense strand sequence
<400> 14
   uucuccgaac gugucacgut tccgttgaca tctcgtagat ttgaatt 47
<210> 15
   <211> 68
   <212> DNA
   <213> Artificial Sequence
<220>
   <223> SSB with 26 base DNA linker sense strand sequence
<400> 15
   ggcugacaac agacuuuaad tdtdcdcdgd tdtdgdadcd adtdcdtdcd gdtdadgdad 60
   tttgaatt 68
<210> 16
   <211> 37
   <212> DNA
   <213> Artificial Sequence
<220>
   <223> Condition #2: SSB with 16 base DNA linker sense strand sequence
<400> 16
   ggcugacaac agacuuuaat tccgttgaca tctcgta 37
<210> 17
   <211> 21
   <212> DNA
   <213> Artificial Sequence
<220>
   <223> Negative Control (no mRNA target) with 16 base linker anti-sense strand sequence
<400> 17
   acgugacacg uucggagaat t 21
<210> 18
   <211> 37
   <212> DNA
   <213> Artificial Sequence
<220>
   <223> Negative Control (no mRNA target) with 16 base linker sense strand sequence
<400> 18
   uucuccgaac gugucacgut tccgttgaca tctcgta 37
<210> 19
   <211> 42
   <212> DNA
   <213> Artificial Sequence
<220>
   <223> Condition #3: SSB with 21 base DNA linker sense strand sequence
<400> 19
   uucuccgaac gugucacgut tccgttgaca tctcgtagat tt 42